# EUROPÄISCHE PATENTSCHRIFT

(11) **EP 3 362 551 B1**
(45) Veröffentlichungstag und Bekanntmachung des Hinweises auf die Patenterteilung: **30.09.2020**
(21) Anmeldenummer: 16763513.5
(22) Anmeldetag: 09.09.2016
(51) Int. Cl.: C12N 1/00, C12N 1/16

(54) **NÄHRMEDIUM FÜR OSMOTOLERANTE MIKROORGANISMEN**
NUTRIENT MEDIUM FOR OSMOTOLERANT MICROORGANISMS
MILIEU DE CULTURE POUR MICRO-ORGANISMES OSMOTOLÉRANTS

(30) Priorität: 14.10.2015 DE 102015117445
(43) Veröffentlichungstag der Anmeldung: 22.08.2018
(73) Patentinhaber: Technische Hochschule Ostwestfalen-Lippe, 32657 Lemgo (DE)
(72) Erfinder: PFANNEBECKER, Jens, 32756 Detmold (DE); BECKER, Barbara, 32105 Bad Salzuflen (DE)
(74) Vertreter: Viering, Jentschura & Partner mbB Patent- und Rechtsanwälte
(86) Internationale Anmeldenummer: PCT/EP2016/071310
(87) Internationale Veröffentlichungsnummer: WO 2017/063797

(56) Entgegenhaltungen:
- PILAR HERNANDEZ ET AL: "Evaluation of diluents and media for enumerating Zygosaccharomyces rouxii in blueberry syrup", INTERNATIONAL JOURNAL OF FOOD MICROBIOLOGY, Bd. 25, Nr. 1, 1. März 1995 (1995-03-01), Seiten 11-18, XP055319142, NL ISSN: 0168-1605, DOI: 10.1016/0168-1605(94)00041-4
- M T Munitis ET AL: "An obligate osmophilic yeast from honey", Applied and Environmental Microbiology, 1. September 1976 (1976-09-01), Seiten 320-323, XP055319166, UNITED STATES Gefunden im Internet: URL:http://aem.asm.org/content/32/3/320.fu ll.pdf [gefunden am 2016-11-15]
- JERMINI M F G ET AL: "DETECTION ISOLATION AND IDENTIFICATION OF OSMOTOLERANT YEASTS FROM HIGH-SUGAR PRODUCTS", JOURNAL OF FOOD PROTECTION, INTERNATIONAL ASSOCIATION FOR FOOD PROTECTION, US , Bd. 50, Nr. 6 1. Juni 1987 (1987-06-01), Seiten 468-472, 478, XP008182254, ISSN: 0362-028X Gefunden im Internet: URL:http://www.ingentaconnect.com/contento ne/iafp/jfp/1987/00000050/00000006/art0000 4#expand/collapse [gefunden am 2016-11-15]
- A D Hocking ET AL: "Dichloran-glycerol medium for enumeration of xerophilic fungi from low-moisture foods", Applied and Environmental Microbiology, 1. März 1980 (1980-03-01), Seiten 488-492, XP055319042, UNITED STATES Gefunden im Internet: URL:http://aem.asm.org/content/39/3/488.fu ll.pdf [gefunden am 2016-11-15]
- R R C ET AL: "Comparison of media for enumerating osmotolerant yeasts in orange juice concentrates", INTERNATIONAL JOURNAL OF FOOD MICROBIOLOGY, ELSEVIER BV, NL, Bd. 22, Nr. 4, 1. Juni 1994 (1994-06-01), Seiten 291-295, XP023851800, ISSN: 0168-1605, DOI: 10.1016/0168-1605(94)90180-5 [gefunden am 1994-06-01]
- L BEUCHAT ET AL: "Performance of mycological media in enumerating desiccated food spoilage yeasts: an interlaboratory study", INTERNATIONAL JOURNAL OF FOOD MICROBIOLOGY, Bd. 70, Nr. 1-2, 22. Oktober 2001 (2001-10-22), Seiten 89-96, XP055319272, NL ISSN: 0168-1605, DOI: 10.1016/S0168-1605(01)00519-0
- JENS PFANNEBECKER ET AL: "Culture medium optimization for osmotolerant yeasts by use of a parallel fermenter system and rapid microbiological testing", JOURNAL OF MICROBIOLOGICAL METHODS, Bd. 130, 1. November 2016 (2016-11-01), Seiten 14-22, XP055319624, NL ISSN: 0167-7012, DOI: 10.1016/j.mimet.2016.08.021

## Beschreibung

### FELD DER ERFINDUNG

Die vorliegende Erfindung liegt im Feld der Mikrobiologie und bezieht sich auf ein Nährmedium zur Kultivierung von osmotoleranten Mikroorganismen und dessen Verwendung. Ferner bezieht sich die vorliegende Erfindung auf ein Verfahren zur Isolierung und/oder Anreicherung von osmotoleranten Mikroorganismen. Des Weiteren umfasst die vorliegende Erfindung ein Kit zur Untersuchung von osmotoleranten Mikroorganismen.

### HINTERGRUND DER ERFINDUNG

Produzenten und Verarbeiter von Lebensmitteln müssen die mikrobiologische Qualität ihrer Rohstoffe und Produkte gemäß gesetzlicher Vorgaben und den Anforderungen an die Produktstabilität gewährleisten. Durch mikrobiologische Kontrollen wird einem möglichen Produktverderb vorgebeugt und der gesundheitliche Verbraucherschutz gewährleistet. Verfahren zum Nachweis von Mikroorganismen in Lebensmitteln sind bekannt und werden von Betriebs-und Dienstleistungslaboren routinemäßig angewandt.

Die mikrobiologische Stabilität von Produkten hängt von vielen Faktoren ab. Neben der Rohstoffqualität spielen vor allem die Wasserverfügbarkeit (a_{w}-Wert), der pH-Wert, das Vorhandensein von Konservierungsmitteln und die Temperatur eine große Rolle. In Rohstoffen und Produkten mit niedrigen a_{w}-Werten (z.B. < 0,95) ist das Wachstum von Mikroorganismen durch die geringe Wasserverfügbarkeit limitiert. Osmotolerante Mikroorganismen können jedoch noch unter diesen Bedingungen wachsen. Produkte wie Flüssigzucker, Marzipan, Nougat, Marmelade, Fruchtkonzentrate, Honig, Trockenfrüchte, Fleisch, Feinkost, Dressings, Ketchup, Mayonnaise, Soja-Sauce und Kondensmilch sind anfällig für eine Kontamination mit diesen Mikroorganismen (Tokuoka, K. (1993), Journal of Applied Bacteriology 74 (2), 101-110; Spencer, J. (2001), NJ: Humana Press (Methods in biotechnology, 14); Deak, T. (2008): Handbook of food spoilage yeasts. 2nd ed. Boca Raton, FL:CRC Press). Vor allem Hefen und Schimmelpilze sind in der Lage in Produkten mit niedrigen a_{w}-Werten zu wachsen. Einige osmotolerante Hefen tolerieren hohe Zucker- bzw. Salzkonzentrationen (z.B. 40-70 % Glucose) und führen zum Verderb von Produkten (Thomas, D.S. (1993): Yeasts as spoilage organisms in beverages. Aus: The Yeasts, A.H. Rose und J.S. Harrison (Eds.). 2.Auflage: Academic Press, New York (5); Schelhorn, M. (1950), Z Lebensm Unters Forch 91 (S), 338-342; Tokuoka, K., lshitani, T. (1991), J. Gen.Appl. Microbiol. 37 (1), 111-119). Zum Beispiel bricht Marzipan auf, geschlossene Verpackungen blähen auf (Bombagenbildung), platzen oder werden undicht (Jermini, M., Geiges, O. und Schmidt-Lorenz, W. (1987), J. Food Prot. (SO), 468-472; Tokuoka, K., lshitani, T., Goto, S. und Komagata, K. (1985), J. Gen.Appl. Microbiol. 31 (5), 411-427). Trübungen können entstehen. Häufig kommt es durch die Bildung von Stoffwechselprodukten wie Essigsäure, Acetaldehyd, Glycerin und Lactat zu Veränderungen der Lebensmittel, die von Konsumenten sensorisch erfassbar sind. Hefen der Gattungen *Zygosaccharomyces, Schizosaccharomyces, Torulaspora, Candida, Wickerhamomyces* und *Debaryomyces* gehören zu den am häufigsten isolierten Schadorganismen aus Rohstoffen und Produkten mit niedrigen a_{w}-Werten und werden oftmals mit Lebensmittelverderb assoziiert (Fleet, G. (1992), Crit Rev Biotechnol 12 (1-2), 1-44). Gasbildung in Verpackungen und sensorische Veränderungen von Rohstoffen und Produkten haben Reklamationen und Rückrufaktionen zur Folge, die beträchtliche finanzielle Einbußen und einen Imageverlust für die betroffenen Unternehmen nach sich ziehen.

Um die Qualität der betreffenden Rohstoffe und Produkte zu sichern, ist ein selektiver und zuverlässiger Nachweis von osmotoleranten Mikroorganismen, insbesondere Hefen, eine entscheidende Voraussetzung. Zur Kultivierung osmotoleranter Hefen finden Nährmedien wie YGC, YPD oder TGYC-Medium (Beuchat, L. (2001), International Journal of Food Microbiology, 70 (1-2), 89-96) Verwendung, die aus Bestandteilen wie Glucose, Hefeextrakt und Pepton zusammengesetzt sind. Das Wachstum von Bakterien wird häufig durch Zugabe von Chloramphenicol unterdrückt. All diesen Nährmedien ist gemein, dass der a_{w}-Wert nicht deutlich abgesenkt ist und somit bei einem Einsatz in der Qualitätskontrolle nicht spezifisch osmotolerante Hefen nachgewiesen werden.

Das in der ISO/FDIS 21527-2 (2008) beschriebene Medium, DG18 (Hocking, A.D., Pitt, J.I. (1980), Appl Environ Microbiol 39 (3), 488-492), besitzt einen a_{w}-Wert von 0,95, der durch das enthaltene Glycerin erreicht wird. Selektivität gegenüber schnell wachsenden Pilzen und Bakterienwachstum wird durch Zugabe von Dichloran und Chloramphenicol erreicht. Vergleichsstudien zeigten, dass DG 18-Agar zum Nachweis von Hefen aus trockenen Matrices mit niedrigen a_{w}-Werten nicht empfohlen werden kann (Beuchat, L. (2001), International Journal of Food Microbiology, 70 (1-2), 89-96).

Boekhout et al. (Boekhout, T., Robert,V. und Phaff, H. (2003): Yeasts in food. Beneficial and detrimental aspects. 1. Aufl. Hamburg: Behr's Verlag), Jermini et al. (Jermini, M., Geiges, O. und Schmidt-Lorenz, W. (1987), J. Food Prot. (SO), 468-472), Combina et al. (Combina, M., Daguerre, C., Massera, A., Mercado, L., Sturm, M. E., Ganga, A. und Martinez, C. (2007): Letters in applied microbiology 46 (2), 192-197) und von Drachenfels (von Drachenfels, H. J. (2009): Süsswaren Technik + Wirtschaft, 54. Jahrgang, 22-23) publizierten Nährmedien mit unterschiedlichen Zuckern und niedrigeren a_{w}-Werten zum Nachweis osmotoleranter Hefen. Diese Nährmedien sind zwar sehr selektiv, jedoch werden Inkubationszeiten von bis zu 21 Tagen benötigt.

In Hernandez et al. (Hernandez, P., Beuchat, L. R. (1995), International Journal of Food Microbiology 25, 11-18) wurden verschiedene Agarzusammensetzungen (DRBC, TGY, DG18, PCA52S, MY50G) in Kombination mit unterschiedlichen Mengen an Glukose (+ Tween 80), Glycerin oder Pepton auf ihr Potential zur Gewinnung von osmotoleranten Mikroorganismen untersucht.

In den lebensmittelproduzierenden und lebensmittelverarbeitenden Unternehmen werden nach eigenen Umfragen sehr verschiedene Untersuchungsverfahren zum Nachweis osmotoleranter Hefen eingesetzt. Viele Betriebe nutzen Standard-Hefenährmedien wie Glucose-Bouillon, Würze-Bouillon oder YGC, die mit a_{w}-Werten > 0,95 nicht ausreichend selektiv für osmotolerante Hefen sind. Die meisten Betriebe führen die Untersuchungen in Bouillonkulturen als qualitativen Test (Presence/Absence-Test) unter Nachweis von Gasbildung durch. Die verwendeten Nährmedien sind hinsichtlich des Gärstoffwechsels der Hefen nicht optimiert. Detektionszeiten schwanken zwischen 10 und 21 Tagen. Die Gasbildung wird oft nicht zuverlässig angezeigt, was einen schnellen und sicheren Nachweis erschwert. Bei Reklamationen von Rohstoffen und Produkten aufgrund von positiven Befunden lassen sich die Ergebnisse häufig nicht verifizieren oder vergleichen. Zusätzliche Kosten, die durch Rücknahme der Ware oder Rechtsstreitigkeiten entstehen, sind die Folge.

Daher besteht gegenwärtig ein Bedürfnis für einen zuverlässigen Nachweis von osmotoleranten Mikroorganismen, insbesondere Hefen, der niedrige Keimzahlen erfasst und der schnell (3-7 Tage) und standardisiert auf viele Spezies anwendbar ist.

### ZUSAMMENFASSUNG DER ERFINDUNG

Gelöst wird die oben beschriebene Aufgabe durch das Nährmedium der vorliegenden Erfindung. Die Erfinder der vorliegenden Erfindung haben überraschend in Vergleichsversuchen festgestellt, dass in einem Nährmedium, das die hier beschriebene erfindungsgemäße Zusammensetzung enthält, für alle untersuchten Spezies osmotoleranter Hefen eine beschleunigte und verstärkte Gäraktivität und CO₂-Transferrate nachgewiesen werden kann. Der verbesserte Nachweis dieser Parameter ermöglicht ein schnelleres und sensitiveres Identifizieren von osmotoleranten Hefen in verschiedenartigen Proben. Das vorliegende erfindungsgemäße Nährmedium ist daher auch für eine Steigerung des Gärstoffwechsels osmotoleranter Hefen optimiert. Im Vergleich zu bestehenden Nährmedien kann das primäre Verderbspotential der Hefen (entspricht der Gasbildung in Produkten) in einem früheren Stadium erkannt werden, was zu einer Zeitersparnis beim Nachweis der Hefen führt.

In einem ersten Aspekt ist die vorliegende Erfindung gerichtet auf ein Nährmedium, umfassend: 15 bis 30 Gew.-% Glucose, vorzugsweise 20 bis 25 Gew.-% Glucose, 0,7 bis 2,0 Gew.-% Fructose, vorzugsweise 1,0 bis 1,3 Gew.-% Fructose, 0,2 bis 0,8 Gew.-% Hefeextrakt, vorzugsweise 0,3 bis 0,5 Gew.-% Hefeextrakt, 0,4 bis 1,5 Gew.-% Malzextrakt, vorzugsweise 0,6 bis 1,0 Gew.-% Malzextrakt, 0,2 bis 0,8 Gew.-% Pepton, vorzugsweise 0,3 bis 0,6 Gew.-% Pepton, 5 bis 15 Gew.-% Glycerin, vorzugsweise 8 bis 12 Gew.-% Glycerin, 2 bis 10 Gew.-% KCl, vorzugsweise 3 bis 5 Gew.-% KCl, 0,04 bis 0,16 Gew.-% MgSO₄, vorzugsweise 0,05 bis 0,1 Gew.-% MgSO₄, 0,04 bis 0,32 Gew.-% (NH₄)₂HPO₄, vorzugsweise 0,1 bis 0,2 Gew.-% (NH₄)₂HPO₄, 0,04 bis 0,16 Gew.-% (NH₄)₂SO₄, vorzugsweise 0,05 bis 0,1 Gew.-% (NH₄)₂SO₄, 0,00003 bis 0,0002 Gew.-% Thiamin, vorzugsweise und 0,00005 bis 0,00015 Gew.-% Thiamin, und 42,2 bis 76,3 Gew.-% Wasser, vorzugsweise 57 bis 67 Gew.-% Wasser.

In verschiedenen Ausführungsformen der Erfindung besteht das erfindungsgemäße Nährmedium aus 21,92 Gew.-% Glucose, 1,15 Gew.-% Fructose, 0,38 Gew.- % Hefeextrakt, 0,77 Gew.-% Malzextrakt, 0,38 Gew.-% Pepton, 9,69 Gew.-% Glycerin, 3,85 Gew.-% KCl, 0,08 Gew.-% MgSO₄, 0,15 Gew.-% (NH₄)₂HPO₄, 0,08 Gew.-% (NH₄)₂SO₄, 0,0001 Gew.-% Thiamin und 61,54 Gew.-% Wasser.

In verschiedenen Ausführungsformen der Erfindung ist der pH-Wert des erfindungsgemäßen Nährmediums zwischen 4,5 und 7,5. Vorzugsweise liegt der pH-Wert zwischen 5,5 und 6,5, in einer weiter bevorzugten Ausführungsform ist der pH-Wert 5,7.

In verschiedenen Ausführungsformen der Erfindung ist der a_{w}-Wert des erfindungsgemäßen Nährmediums zwischen 0,86 und 0,95. Vorzugsweise liegt der a_{w}-Wert zwischen 0,88 und 0,92, in einer weiter bevorzugten Ausführungsform ist der a_{w}-Wert 0,90.

In verschiedenen Ausführungsformen der Erfindung ist das Nährmedium ein festes Nährmedium, das vorzugsweise Agar enthält. In verschiedenen Ausführungsformen umfasst der Hefeextrakt 5-40 Gew.-% Proteine, 5-40 Gew.-% freie Aminosäuren und 0,001-2 Gew.-% Vitamine der B-Gruppe oder besteht aus diesen. In verschiedenen Ausführungsformen umfasst der Malzextrak 55-80 Gew.-% Kohlenhydrate, insbesondere Maltose, Glucose, Fructose, Saccharose, 1-12 Gew.-% stickstoffhaltige Verbindungen, insbesondere Peptide, Aminosäuren, Purine, Vitamine, oder besteht aus Maltose.

Ein zweiter Aspekt der Erfindung ist gerichtet auf ein Verfahren zur Isolierung und/oder Anreicherung von osmotoleranten Mikroorganismen, umfassend: Kultivieren einer Probe eines osmotoleranten Mikroorganismus in einem erfindungsgemäßen Nährmedium.

In verschiedenen Ausführungsformen der Erfindung ist der osmotolerante Mikroorganismus eine Hefe, vorzugsweise eine Hefe ausgewählt aus der Gattung *Zygosaccharomyces, Schizosaccharomyces, Torulaspora, Candida, Wickerhamomyces, Pichia, Starmerella* und *Debaryomyces.*

In verschiedenen Ausführungsformen der Erfindung ist die Probe entnommen oder ausgewählt aus Nahrungsmitteln, vorzugsweise Flüssigzucker, Marzipan, Persipan, Schokoladenprodukt, Nougat, Honig, Sirup (z. B. Invertzuckersirup, Glucosesirup, Fructosesirup, Melasse), Zuckerdicksaft, Fruchtkonzentrat, Konfitüre, Limonade, Fleischprodukt, Feinkostsalate, Kondensmilch, Trockenfrüchte oder Sojasauce, Futtermitteln, pharmazeutischen Produkten, Kosmetika und klinisch-diagnostischen Proben.

In einem dritten Aspekt ist die vorliegende Erfindung gerichtet auf eine Verwendung des erfindungsgemäßen Nährmediums zur Kultivierung von osmotoleranten Mikroorganismen, vorzugsweise osmotolerante Hefen.

Ein vierter Aspekt der Erfindung ist gerichtet auf ein Kit zur Untersuchung von osmotoleranten Mikroorganismen, umfassend: das erfindungsgemäße Nährmedium, Kulturgefäß und eine Vorrichtung zum Nachweis der Gasbildung der osmotoleranten Mikroorganismen.

### KURZE BESCHREIBUNG DER ABBILDUNGEN

Nachstehend wird die Erfindung anhand von mehreren Abbildungen näher erläutert. Diese unterstützen die in der Beschreibung erläuterte Lehre, schränken diese aber nicht ein. Hierbei zeigt:

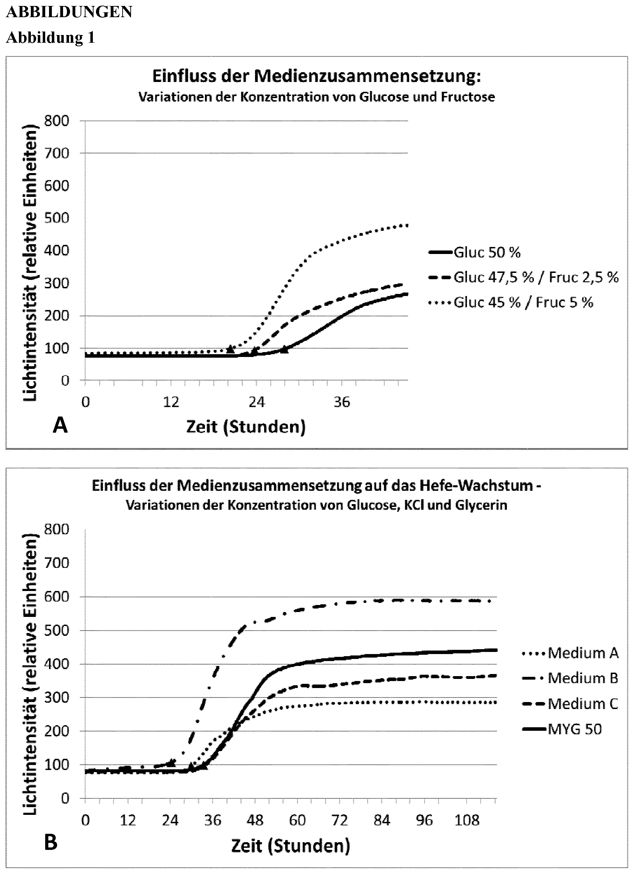

**Abbildung 1****:** Einfluss der Medienzusammensetzung auf das Wachstum osmotoleranter Hefen im BioLumix™-System. A) Einfluss der Zusammensetzung aus Glucose und Fructose (a_{w}-Werte 0,90). B) Einfluss der Zusammensetzung aus Glucose, KCl und Glycerin (Konzentrationen der Ansätze "A, B, C" siehe Tab. 4). Untersucht wurde Z. *rouxii* (WH 1002). Inokulum: 2,1 · 10³ KbE / ml. Die Lichtintensität ist in relativen Einheiten angegeben. Dreiecke geben den Zeitpunkt der Detektion im BioLumix™-System an.

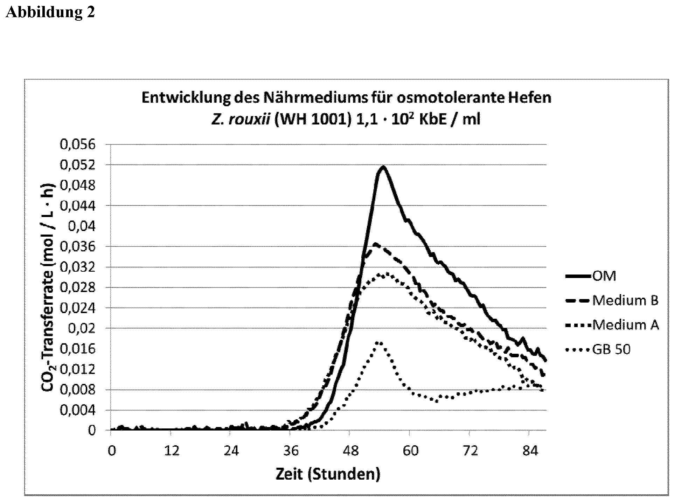

**Abbildung 2****:** Vergleich der CO₂-Transferrate osmotoleranter Hefen in GB 50, den erfindungsgemäßen Nährmedien Medium A und Medium B und dem erfindungsgemäßen Nährmedium OM im RAMOS®-Parallelfermenter (HiTec Zang GmbH, Herzogenrath). Fermentationsansatz mit *Zygosaccharomyces rouxii* (WH 1001), Inokulum je 1,1 · 10² KbE/ml.

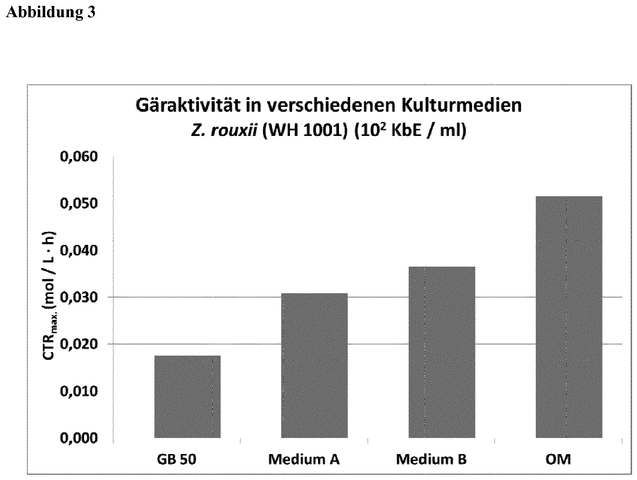

**Abbildung 3****:** Vergleich der maximalen Gäraktivität (CTRₘₐₓ) osmotoleranter Hefen in GB 50, Medium A, Medium B und im Nährmedium OM im RAMOS®-Parallelfermenter (HiTec Zang GmbH, Herzogenrath). *Zygosaccharomyces rouxii* (WH 1001). Ausgangskeimzahlen: je 10¹ - 10² KbE / ml. Die angegebenen Werte sind Mittelwerte aus Doppelbestimmungen.

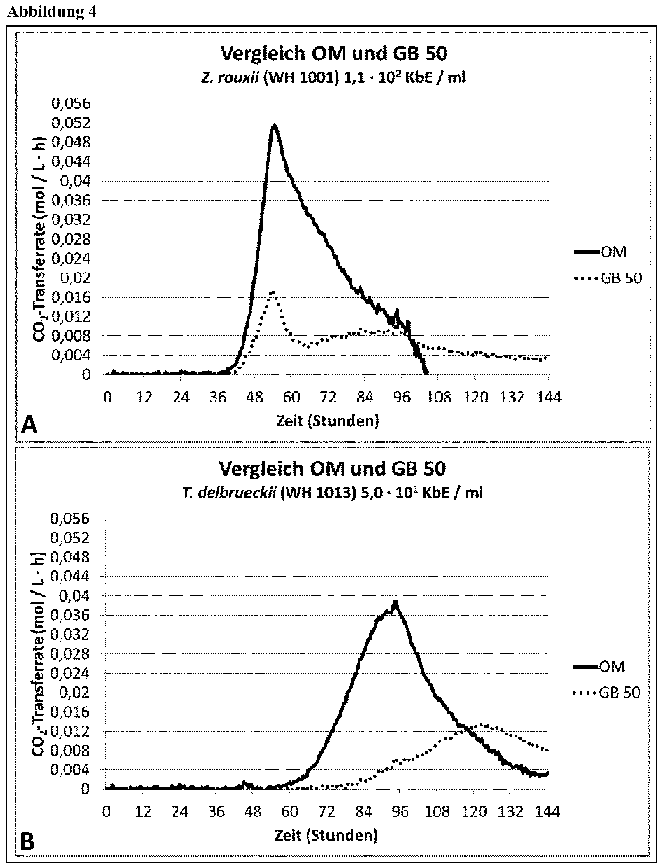

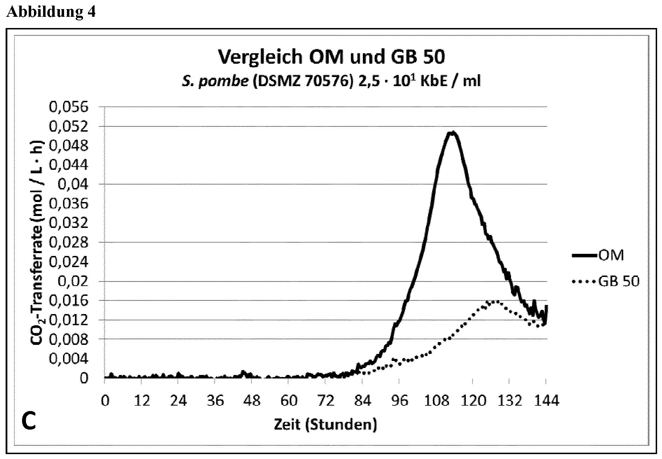

**Abbildung 4****:** Vergleich der CO₂-Transferrate osmotoleranter Hefen in GB 50 (Nährmedium aus dem Stand der Technik) und dem erfindungsgemäßen Nährmedium (hier: "OM") im RAMOS®-Parallelfermenter (HiTec Zang GmbH, Herzogenrath). A: Fermentationsansatz mit *Zygosaccharomyces rouxii* (WH 1001), Inokulum je 1,1 · 10² KbE/ml; B: Fermentationsansatz mit *Torulaspora delbrueckii* (WH 1013), Inokulum je 5,0 · 10¹ KbE/ml; C: Fermentationsansatz mit *Schizosaccharomyces pombe* (DSMZ 70576), Inokulum je 2,5 · 10¹ KbE/ml.

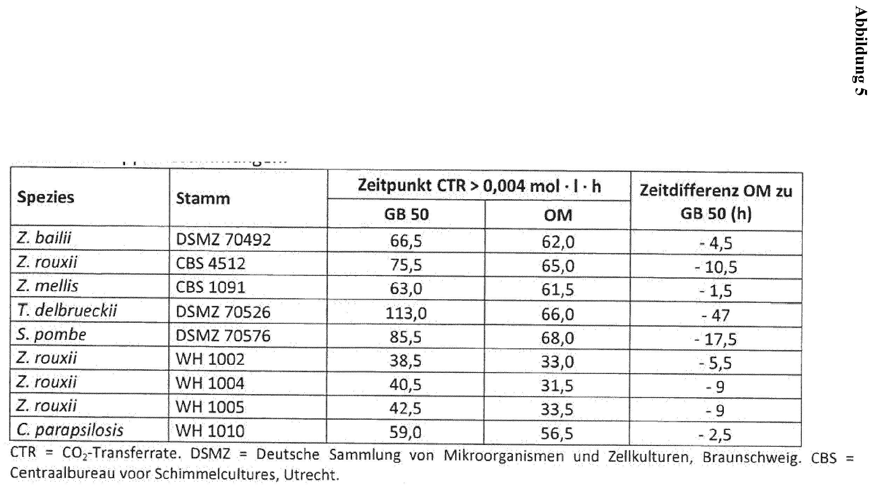

**Abbildung 5****:** Vergleich von Nachweiszeiten bis zum Einsetzen der Gärung CO₂-Transferrate (CTR > 0,004 mol / l · h) in GB 50 und dem erfindungsgemäßen Nährmedium OM im RAMOS®-Parallelfermenter (HiTec Zang GmbH, Herzogenrath). Angegeben sind Mittelwerte aus Doppelbestimmungen.

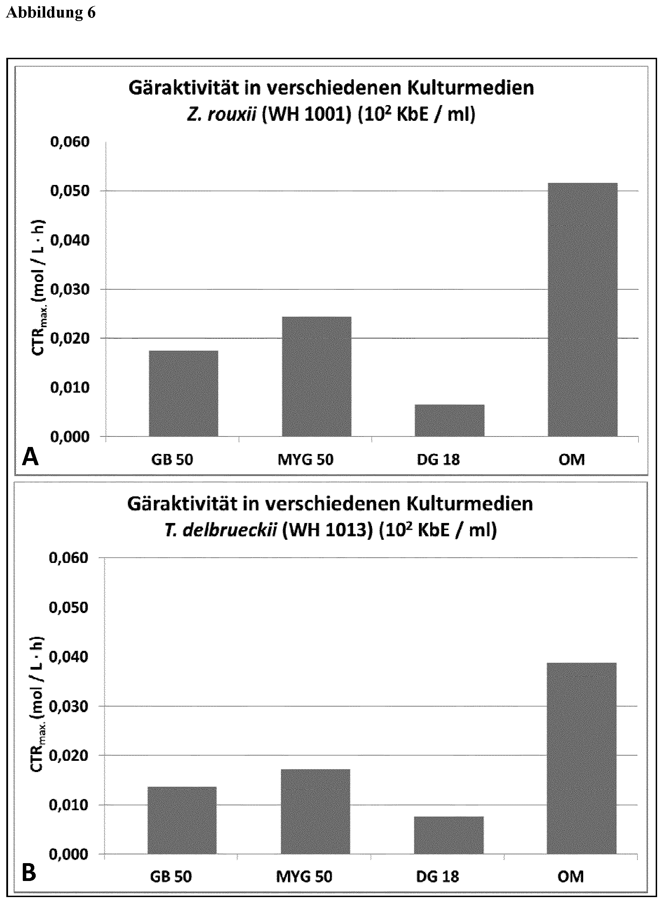

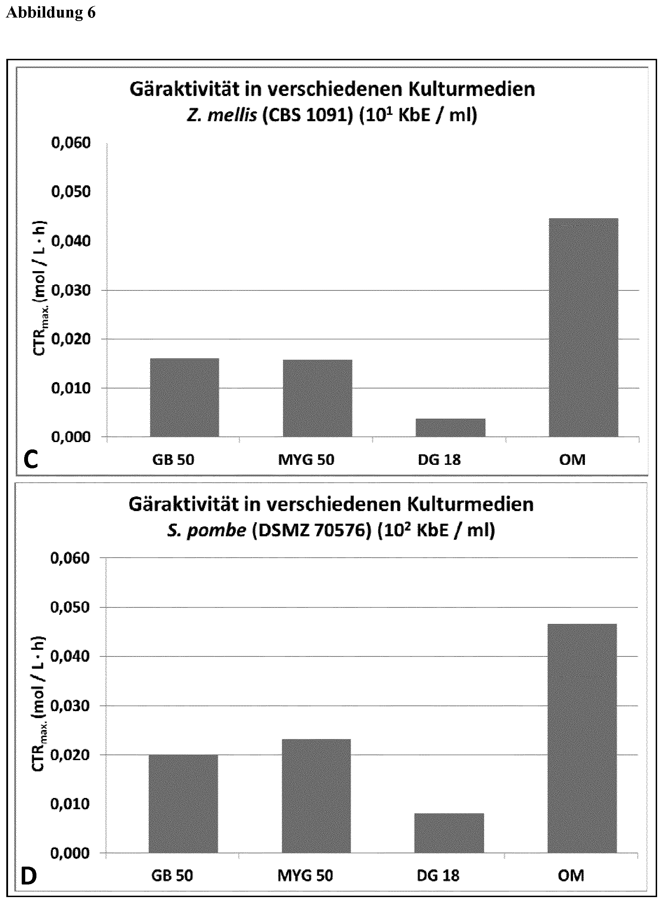

**Abbildung 6****:** Vergleich der maximalen Gäraktivität (CTR_{max.}) osmotoleranter Hefen in Nährmedien des Standes der Technik (GB 50, MYG 50, DG 18) und im erfindungsgemäßen Nährmedium (OM) im RAMOS®-Parallelfermenter (HiTec Zang GmbH, Herzogenrath). A: *Zygosaccharomyces rouxii* (WH 1001), B: *Torulaspora delbrueckii* (WH 1013). C: *Zygosaccharomyces mellis* (CBS 1091), D: *Schizosaccharomyces pombe* (DSM 70576). Ausgangskeimzahlen: je 10¹ - 10² KbE / ml. Die angegebenen Werte sind Mittelwerte aus Doppelbestimmungen.

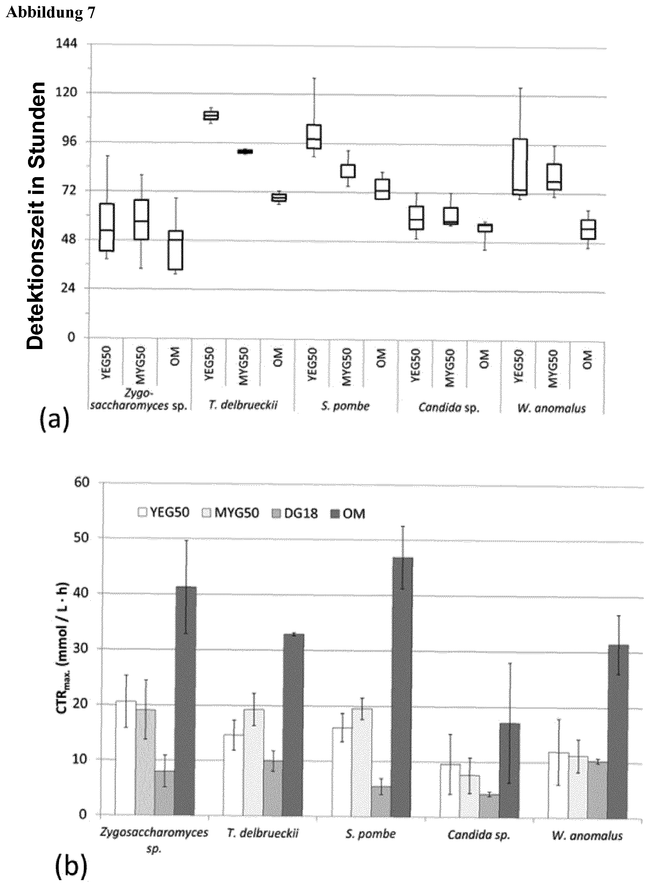

**Abbildung 7****:** Boxplots zeigen die Detektionszeiten (Zeit bis CTR ≥ 4 mmol / l · h) (a) und den Vergleich von maximalen CO₂-Transferraten (CTR_{max.}) (b) von 9 *Zygosaccharomyces* Stämmen, 4 *Candida* Stämmen, 2 Stämmen der Spezies *Torulaspora delbrueckii*, 5 *Schizosaccharomyces pombe* Stämmen und 3 *Wickerhamomyces anomalus* Stämmen in GB 50, MYG 50 und OM Bouillon analysiert bei initialen Keimzahlen von 10² KbE/ml im RAMOS Parallelfermenter.

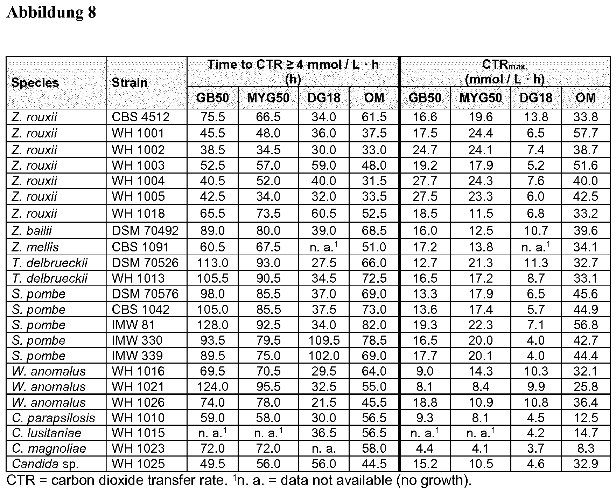

**Abbildung 8****:** Zeit bis zum Beginn der Fermentation (CO₂-Transferrate (CTR) ≥ 4 mmol / l · h) und der maximalen CO₂-Transferrate (CTR_{max.}) von osmotoleranten Hefen, die in GB 50, MYG 50, DG18 und OM Kulturmedium im RAMOS Parallelfermenter untersucht wurden. Inokulum: 10² KbE/ml.

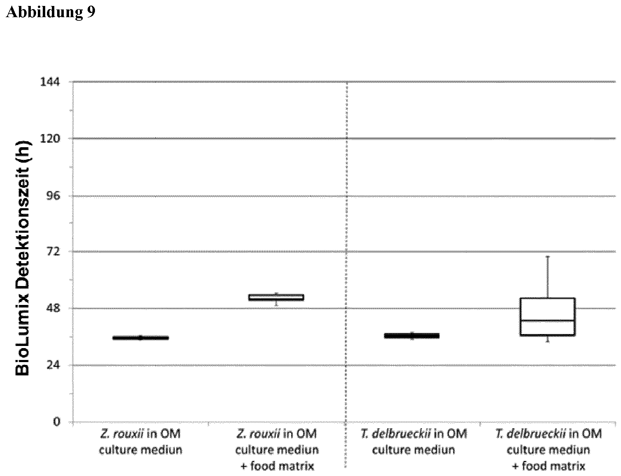

**Abbildung 9****:** Ein Boxplot zeigt die Detektionszeiten für *Zygosaccharomyces rouxii* (WH 1001) und *Torulaspora delbrueckii* (WH 1013) im Biolumix™ System bei initialen Keimzahlen zwischen 10¹ und 10² KbE/ml in OM Kulturmedium und in OM Kulturmedium mit stark zuckerhaltigen Lebensmittel-Matrices im Verhältnis von 1:10. Die Daten basieren auf der Prüfung der folgenden Lebensmittel: Marzipanrohmasse, Nougatfüllung, granulierter Honig, Zuckersirup, Persipanpaste, Mohnfüllung und Erdbeermarmelade. Die Bestimmung erfolgte in Duplikaten.

### DETAILLIERTE BESCHREIBUNG DER ERFINDUNG

Die Erfinder der vorliegenden Erfindung haben überraschend die Zusammensetzung für ein Nährmedium gefunden, das einen zuverlässigen und selektiven Nachweis von osmotoleranten Mikroorganismen, insbesondere osmotoleranten Hefen, erlaubt und das den Nachweis von niedrigen Keimzahlen schnell (3-7 Tage) und für viele Spezies ermöglicht.

Daher ist in einem ersten Aspekt die vorliegende Erfindung gerichtet auf ein Nährmedium, umfassend: 15 bis 30 Gew.-% Glucose, vorzugsweise 20 bis 25 Gew.-% Glucose, 0,7 bis 2,0 Gew.-% Fructose, vorzugsweise 1,0 bis 1,3 Gew.-% Fructose, 0,2 bis 0,8 Gew.-% Hefeextrakt, vorzugsweise 0,3 bis 0,5 Gew.-% Hefeextrakt, 0,4 bis 1,5 Gew.-% Malzextrakt, vorzugsweise 0,6 bis 1,0 Gew.-% Malzextrakt, 0,2 bis 0,8 Gew.-% Pepton, vorzugsweise 0,3 bis 0,6 Gew.-% Pepton, 5 bis 15 Gew.-% Glycerin, vorzugsweise 8 bis 12 Gew.-% Glycerin, 2 bis 10 Gew.-% KCl, vorzugsweise 3 bis 5 Gew.-% KCl, 0,04 bis 0,16 Gew.-% MgSO₄, vorzugsweise 0,05 bis 0,1 Gew.-% MgSO₄, 0,04 bis 0,32 Gew.-% (NH₄)₂HPO₄, vorzugsweise 0,1 bis 0,2 Gew.-% (NH₄)₂HPO₄, 0,04 bis 0,16 Gew.-% (NH₄)₂SO₄, vorzugsweise 0,05 bis 0,1 Gew.-% (NH₄)₂SO₄, 0,00003 bis 0,0002 Gew.-% Thiamin, vorzugsweise 0,00005 bis 0,00015 Gew.-% Thiamin, und 42,2 bis 76,3 Gew.-% Wasser, vorzugsweise 57 bis 67 Gew.-% Wasser.

In verschiedenen Ausführungsformen umfasst das Nährmedium 15 bis 30 Gew.-% Glucose, vorzugsweise 20 bis 25 Gew.-% Glucose, 0,7 bis 2,0 Gew.-% Fructose, vorzugsweise 1,0 bis 1,3 Gew.-% Fructose, 0,2 bis 0,8 Gew.-% Hefeextrakt, vorzugsweise 0,3 bis 0,5 Gew.-% Hefeextrakt, 5 bis 15 Gew.-% Glycerin, vorzugsweise 8 bis 12 Gew.-% Glycerin, 2 bis 10 Gew.- % KCl, vorzugsweise 3 bis 5 Gew.-% KCl, 0,04 bis 0,16 Gew.-% MgSO₄, vorzugsweise 0,05 bis 0,1 Gew.-% MgSO₄, 0,04 bis 0,32 Gew.-% (NH₄)₂HPO₄, vorzugsweise 0,1 bis 0,2 Gew.-% (NH₄)₂HPO₄, 0,00003 bis 0,0002 Gew.-% Thiamin, vorzugsweise und 0,00005 bis 0,00015 Gew.-% Thiamin und 47,72 bis 76,3 Gew.-% Wasser, vorzugsweise 55,9 bis 67,5 Gew.-% Wasser.

In verschiedenen Ausführungsformen umfasst das Nährmedium 15 bis 30 Gew.-% Glucose, vorzugsweise 20 bis 25 Gew.-% Glucose, 0,7 bis 2,0 Gew.-% Fructose, vorzugsweise 1,0 bis 1,3 Gew.-% Fructose, 0,2 bis 0,8 Gew.-% Hefeextrakt, vorzugsweise 0,3 bis 0,5 Gew.-% Hefeextrakt, 5 bis 15 Gew.-% Glycerin, vorzugsweise 8 bis 12 Gew.-% Glycerin, 2 bis 10 Gew.- % KCl, vorzugsweise 3 bis 5 Gew.-% KCl, 0,04 bis 0,16 Gew.-% MgSO₄, vorzugsweise 0,05 bis 0,1 Gew.-% MgSO₄, 0,04 bis 0,32 Gew.-% (NH₄)₂HPO₄, vorzugsweise 0,1 bis 0,2 Gew.-% (NH₄)₂HPO₄, 0,00003 bis 0,0002 Gew.-% Thiamin, vorzugsweise und 0,00005 bis 0,00015 Gew.-% Thiamin und 47,72 bis 76,3 Gew.-% Wasser, vorzugsweise 55,9 bis 67,5 Gew.-% Wasser.

In verschiedenen Ausführungsformen besteht das Nährmedium aus 21,92 Gew.-% Glucose, 1,15 Gew.-% Fructose, 0,38 Gew.-% Hefeextrakt, 9,69 Gew.-% Glycerin, 3,85 Gew.-% KCl, 0,08 Gew.-% MgSO₄, 0,15 Gew.-% (NH₄)₂HPO₄, 0,0001 Gew.-% Thiamin und 62,78 Gew.-% Wasser.

In verschiedenen Ausführungsformen umfasst das Nährmedium 15 bis 30 Gew.-% Glucose, vorzugsweise 20 bis 25 Gew.-% Glucose, 0,7 bis 2,0 Gew.-% Fructose, vorzugsweise 1,0 bis 1,3 Gew.-% Fructose, 0,2 bis 0,8 Gew.-% Hefeextrakt, vorzugsweise 0,3 bis 0,5 Gew.-% Hefeextrakt, 0,2 bis 0,8 Gew.-% Pepton, vorzugsweise 0,3 bis 0,6 Gew.-% Pepton, 5 bis 15 Gew.-% Glycerin, vorzugsweise 8 bis 12 Gew.-% Glycerin, 2 bis 10 Gew.-% KCl, vorzugsweise 3 bis 5 Gew.-% KCl, 0,04 bis 0,16 Gew.-% MgSO₄, vorzugsweise 0,05 bis 0,1 Gew.-% MgSO₄, 0,04 bis 0,32 Gew.-% (NH₄)₂HPO₄, vorzugsweise 0,1 bis 0,2 Gew.-% (NH₄)₂HPO₄, 0,00003 bis 0,0002 Gew.-% Thiamin, vorzugsweise und 0,00005 bis 0,00015 Gew.-% Thiamin und 40,9 bis 76,3 Gew.-% Wasser, vorzugsweise 55,3 bis 67,3 Gew.-% Wasser.

In verschiedenen Ausführungsformen besteht das Nährmedium aus 21,92 Gew.-% Glucose, 1,15 Gew.-% Fructose, 0,38 Gew.-% Hefeextrakt, 0,38 Gew.-% Pepton, 9,69 Gew.-% Glycerin, 3,85 Gew.-% KCl, 0,08 Gew.-% MgSO₄, 0,15 Gew.-% (NH₄)₂HPO₄, 0,0001 Gew.-% Thiamin und 62,4 Gew.-% Wasser.

In verschiedenen Ausführungsformen der Erfindung umfasst das erfindungsgemäße Nährmedium 15 bis 30 Gew.-% Glucose, vorzugsweise 20 bis 25 Gew.-% Glucose, 0,7 bis 2,0 Gew.-% Fructose, vorzugsweise 1,0 bis 1,3 Gew.-% Fructose, 0,2 bis 0,8 Gew.-% Hefeextrakt, vorzugsweise 0,3 bis 0,5 Gew.-% Hefeextrakt, 0,4 bis 1,5 Gew.-% Malzextrakt, vorzugsweise 0,6 bis 1,0 Gew.-% Malzextrakt, 0,2 bis 0,8 Gew.-% Pepton, vorzugsweise 0,3 bis 0,6 Gew.-% Pepton, 5 bis 15 Gew.-% Glycerin, vorzugsweise 8 bis 12 Gew.-% Glycerin, 2 bis 10 Gew.-% KCl, vorzugsweise 3 bis 5 Gew.-% KCl, 0,04 bis 0,16 Gew.- % MgSO₄, vorzugsweise 0,05 bis 0,1 Gew.-% MgSO₄, 0,04 bis 0,32 Gew.-% (NH₄)₂HPO₄, vorzugsweise 0,1 bis 0,2 Gew.-% (NH₄)₂HPO₄, 0,04 bis 0,16 Gew.-% (NH₄)₂SO₄, vorzugsweise 0,05 bis 0,1 Gew.-% (NH₄)₂SO₄, 0,00003 bis 0,0002 Gew.-% Thiamin, vorzugsweise und 0,00005 bis 0,00015 Gew.-% Thiamin und 55 bis 76,3 Gew.-% Wasser, vorzugsweise 57 bis 67 Gew.-% Wasser.

In verschiedenen Ausführungsformen der Erfindung besteht das erfindungsgemäße Nährmedium aus 21,92 Gew.-% Glucose, 1,15 Gew.-% Fructose, 0,38 Gew.- % Hefeextrakt, 0,77 Gew.-% Malzextrakt, 0,38 Gew.-% Pepton, 9,69 Gew.-% Glycerin, 3,85 Gew.-% KCl, 0,08 Gew.-% MgSO₄, 0,15 Gew.-% (NH₄)₂HPO₄, 0,08 Gew.-% (NH₄)₂SO₄, 0,0001 Gew.-% Thiamin und 61,54 Gew.-% Wasser.

In den Ausführungsformen, in denen die einzelnen Komponenten des erfindungsgemäßen Nährmediums als Wertebereich in Gewichtsprozent angegeben sind, ist für die jeweilige Ausführungsform die Summe aller Gewichtsprozente der einzelnen Komponenten nicht höher als 100%.

Der Begriff "Nährmedium" oder "Kulturmedium", wie hierin verwendet, bezeichnet ein Substrat, das zur Kultivierung von Mikroorganismen, Zellen, Geweben oder kleinen Pflanzen dient. Man unterscheidet zwischen flüssigen (zum Beispiel Bouillon, Nährbouillon oder Nährlösung) und gelierten ("festen") Nährmedien (Nährboden). Bei Festmedien steht die Isolierung und Analyse der jeweiligen Mikroorganismen im Vordergrund, daher können sie auch als Isolierungsmedien bezeichnet werden. Bei der Quantifizierung von Mikroorganismen verwendet man feste Medien zur Bestimmung der Koloniezahl. In einer bevorzugten Ausführungsform der Erfindung ist das erfindungsgemäße Nährmedium ein festes Nährmedium. In einer noch bevorzugteren Ausführungsform wird das feste Nährmedium durch das Geliermittel Agar verfestigt, wobei Agar, oder auch Agar-Agar, ein Galactose-Polymer (ein Polysaccharid) ist, das Gallerte bilden kann. Die Grundeinheiten des Agars sind Agarose und sulfatiertes Agaropektin. Agar wird aus den Zellwänden einiger Algenarten (vor allem Rotalgen, wie *Gracilaria*-, *Gelidiopsis-, Gelidium*-, *Hypnea-* und *Sphaerococcus-Arten*), hauptsächlich aus Ostasien, hergestellt.

Im Gegensatz zu festen Nährmedien werden Flüssigmedien zumeist als Anreicherungsmedium verwendet. Ebenso werden sie genutzt, wenn man größere Mengen einer Kultur benötigt. Bei der Quantifizierung von Mikroorganismen verwendet man flüssige Medien im Titerverfahren.

Der Begriff "Glucose", wie hierin verwendet, bezeichnet ein Monosaccharid (Einfachzucker) der Summenformel C₆H₁₂O₆, das damit zu den Kohlenhydraten gehört. Es gibt zwei Enantiomere: D-Glucose und L-Glucose. Wenn in der vorliegenden Erfindung der Begriff "Glucose" verwendet wird bezieht er sich ohne Einschränkung auf beide Enantiomere. In verschiedenen Ausführungsformen der Erfindung ist D-Glucose bevorzugt. In der Natur kommt ausschließlich D-Glucose vor. Diese wird auch als Traubenzucker oder in älterer Literatur als Dextrose bezeichnet. Glucose liegt in fester Form meist als Monohydrat Hydratdextrose vor. L-Glucose ist nur synthetisch zugänglich.

"Fructose", wie hierin verwendet, ist eine natürlich vorkommende chemische Verbindung mit der Summenformel C₆H₁₂O₆. Fructose gehört als Monosaccharid zu den Kohlenhydraten. Sie kommt in mehreren isomeren (anomeren) Formen vor, insbesondere D-Fructose, L-Fructose und DL-Fructose. Wenn in der vorliegenden Erfindung der Begriff "Fructose" verwendet wird bezieht er sich ohne Einschränkung auf alle Enantiomere. In verschiedenen Ausführungsformen der Erfindung ist D-Fructose bevorzugt.

Der Begriff "Hefeextrakt", wie hierin verwendet, bezieht sich auf ein Konzentrat der löslichen Inhaltsstoffe von Hefezellen. Es handelt sich um eine braune Paste oder ein sprühgetrocknetes gelbbräunliches, wasserlösliches Pulver. Hefeextrakt enthält einen hohen Anteil an Proteinen, Aminosäuren und Vitaminen der B-Gruppe. Hefeextrakt kann neben seiner Verwendung als Komponente in Nährmedien auch als Brotaufstrich, Würzmittel und Geschmacksverstärker sowie, vergleichbar mit Nährhefe, in der Krankenernährung verwendet werden. In verschiedenen Ausführungsformen umfasst der Hefeextrakt 5-40 Gew.-% Proteine, 5-40 Gew.-% freie Aminosäuren und 0,001-2 Gew.-% Vitamine der B-Gruppe oder besteht aus diesen.

"Malzextrakt", wie hierin verwendet, ist ein aus Gersten-Malz hergestellter, hellbis dunkelbrauner, malzartig schmeckender Sirup, der neben seiner Verwendung als Komponente in Nährmedien unter anderem auch als Backmittel verwendet werden kann. Der Maltosegehalt beträgt mindestens 55 %, der Dextringehalt höchstens 12 %. Die wichtigsten Aromasubstanzen sind Maltol und 3-Methylbutanal. Getrockneter Malzextrakt (Sprühtrocknung → 98 % Trockenmasse) wird z. B. in Form von Liebigs Malzextrakt als Stärkungsmittel angeboten. In verschiedenen Ausführungsformen umfasst der Malzextrakt 55-80 Gew.-% Kohlenhydrate, insbesondere Maltose (≥ 55 Gew.-%), Glucose, Fructose, Saccharose, 1-12 Gew.-% stickstoffhaltige Verbindungen, insbesondere Peptide, Aminosäuren, Purine, Vitamine, oder besteht aus Maltose.

Der Begriff "Pepton", wie hierin verwendet, bezeichnet ein Gemisch aus Peptiden und Aminosäuren, das durch Hydrolyse durch das Enzym Pepsin oder chemisch durch Säuren aus tierischen oder pflanzlichen Proteinen hergestellt wird. Peptone besitzen nur eine geringe Molekülmasse, wodurch sie nicht durch Salz ausgefällt werden oder verklumpen ("koagulieren"). Dadurch bleiben sie gelöst und können zum Beispiel in Nährlösungen für Mikroorganismen eingesetzt werden, beispielsweise im Casein-Soja-Pepton-Agar. Im Gegensatz dazu werden tryptisch verdaute Proteine (mittels Trypsin) als Trypton bezeichnet. In einer bevorzugten Ausführungsform der Erfindung ist das Pepton ein Pepton aus Fleisch oder ein Casein-Pepton.

"Glycerin", wie hierin verwendet, ist ein Trivialname der Bezeichnung Propan-1,2,3-triol. Glycerin ist ein Zuckeralkohol und der einfachste dreiwertige Alkohol, ein Triol. Glycerin ist in allen natürlichen Fetten und fetten Ölen als Fettsäureester (Triglyceride) vorhanden und spielt eine zentrale Rolle als Zwischenprodukt in verschiedenen Stoffwechselprozessen.

Der Begriff "Thiamin", wie hierin verwendet, oder auch Vitamin B1 oder Aneurin ist ein wasserlösliches Vitamin aus dem B-Komplex von schwachem, aber charakteristischem Geruch. Thiamin besteht aus zwei Ringsystemen, die durch eine Methylenbrücke miteinander verbunden sind: einem Pyrimidin- und einem Thiazolring. Für Thiamin ist bekannt, dass es in Form von Thiaminpyrophosphat (TPP) als Cofaktor der Pyruvat-Decarboxylase die Gärung beschleunigt.

Ammonium besitzt auf die Zellvermehrung und den Gärstoffwechsel der Hefen einen positiven Einfluss (Colombie, S. et al. (2007), J Biosci Bioeng 103 (3), 229-235). Besonders in der stationären Phase des Hefewachstums führt die Verfügbarkeit von Ammonium zu einer höheren Fermentationsrate (Mendes-Ferreira, A. et al. (2004), J Appl Microbiol 97 (3), 540-545; Schnierda, T. et al. (2014), Letters in applied microbiology 58 (5), 478-485).

Der Begriff "wasseraktive Substanz", wie hierin verwendet, bezieht sich auf ein wasserlösliches Salz wie Kaliumchlorid, Magnesiumsulfat, Magnesiumchlorid, Kaliumsulfat, Natriumchlorid, Sorbitol, Inositol, Urea oder ein wasseranziehendes Polymer wie beispielsweise Polyethylenglycol (PEG) mit einem molekularen Gewicht von 500 bis 20.000 oder ein Poly(hydroxyalkylmethacrylat) mit einem molekularen Gewicht von 30.000 bis 5.000.000 oder ein Poly(vinylpyrrolidin) mit einem molekularen Gewicht von 10.000 bis 360.000, ein anionisches oder kationisches Hydrogel oder ein Polyvinyl-Alkohol, der einen geringen Acetatrest hat.

In verschiedenen Ausführungsformen der vorliegenden Erfindung ist die Nährmediumskomponente "Wasser" destilliertes Wasser oder entmineralisiertes Wasser (VE-Wasser).

In verschiedenen Ausführungsformen beträgt der pH-Wert des erfindungsgemäßen Nährmediums zwischen 4,5 und 7,5. In einer weiter bevorzugten Ausführungsform beträgt der pH-Wert 5,7. Der pH-Wert ist ein Maß für den sauren oder basischen Charakter einer wässrigen Lösung. Er ist der negative dekadische Logarithmus der [H⁺]-Konzentration geteilt durch die Standardkonzentration. Der pH-Wert ist eine dimensionslose Zahl.

Der "a_{w}-Wert", wie hierin verwendet, oder synonym "Wasseraktivität" ist ein Maß für frei verfügbares Wasser in einem Material. Er ist definiert als Quotient des Wasserdampfdrucks über einem Material (p) zu dem Wasserdampfdruck über reinem Wasser (po) bei einer bestimmten Temperatur (a_{w} = p/p₀). Der a_{w}-Wert ist ein wichtiges Maß bezüglich der Haltbarkeit von Lebensmitteln und beeinflusst das Vorkommen der Mikroorganismen (Verderbniserreger), die unterschiedliche Ansprüche an frei verfügbares Wasser haben. Bei Mangel an freiem Wasser werden die Wachstumsprozesse von einigen Mikroorganismen verlangsamt, empfindliche Organismen können abgetötet werden. Osmotolerante Organismen hingegen können bei sinkendem Wassergehalt noch Stoffwechsel betreiben und sich ggf. vermehren. Bei den meisten Mikroorganismen liegt das Wachstumsoptimum bei einem a_{w}-Wert von 0,98 bis 1. Es gibt jedoch Mikroorganismen, die eine deutlich niedrigere Wasseraktivität von bis zu 0,6 tolerieren (so genannte xerophile). Beispiele sind osmotolerante (zuckerliebende) Hefen oder extrem halophile Bakterien. In verschiedenen Ausführungsformen beträgt der a_{w}-Wert des erfindungsgemäßen Nährmediums zwischen 0,88 und 0,92. In einer weiter bevorzugten Ausführungsform beträgt der a_{w}-Wert 0,90.

In einem zweiten Aspekt ist die vorliegende Erfindung gerichtet auf ein Verfahren zur Isolierung und/oder Anreicherung von osmotoleranten Mikroorganismen, umfassend: Kultivieren einer Probe eines osmotoleranten Mikroorganismus in einem erfindungsgemäßen Nährmedium. Das Isolieren von osmotoleranten Mikroorganismen, wie hierin verwendet, bezieht sich auf das Abtrennen der osmotoleranten Mikroorganismen von anderen Organismen und/oder Bestandteile dieser Organismen. Ein gemeinsames Vorkommen von osmotoleranten Mikroorganismen und anderen Organismen in einer Probe kann dadurch entstehen, dass diese Organismen dasselbe Habitat bevorzugen. Es ist allerdings auch möglich, dass eine Probe von osmotoleranten Mikroorganismen nach ihrem Entnehmen durch andere Organismen kontaminiert wird. Das Isolieren im Sinne der Erfindung bezieht sich auf ein Vereinzeln des osmotoleranten Mikroorganismus (100% Reinheit) sowie auf ein Abtrennen von bis zu 40%, bis zu 50%, bis zu 60%, bis zu 70%, bis zu 80%, bis zu 90%, bis zu 95% oder bis zu 99% aller nicht-osmotoleranten Organismen, vorzugsweise aller nicht-osmotoleranten Hefen.

Der Begriff "Anreichern", wie hierin verwendet, kann sich auf das Wachstum eines einzelnen Individuums (Gewichtszunahme) und/oder der Vermehrung einer Population von Organismen beziehen. In bevorzugten Ausführungsformen bezieht sich der Begriff "Anreichern" auf die Vermehrung einzelliger Hefen durch Zellteilung, insbesondere Sprossung/Knospung.

"Osmotoleranz" oder "osmotolerant", wie hierin verwendet, bezieht sich auf die Fähigkeit von Mikroorganismen hohen Konzentrationen an gelösten Substanzen in wässerigen Lösungen Stand zu halten, d.h. in einem solchen Medium überleben und sich ggf. vermehren zu können. Die gelösten Substanzen können insbesondere gelöste Zucker oder Salze sein. Unter den Begriff "Mikroorganismus", wie hierin verwendet, sind mikroskopisch kleine Lebewesen (Organismen) ungleicher Abstammung zusammen zu verstehen, die vergleichsweise einfach strukturiert sind. Mikroskopisch klein heißt, dass man diese Lebewesen in aller Regel mit bloßem Auge nicht sehen kann. Meist handelt es sich bei Mikroorganismen um Bakterien, Schimmelpilze oder Hefen. Zu den Mikroorganismen im Sinne der Erfindung gehören weiterhin bestimmte Pflanzen, Tiere und Pilze.

"Kultivieren" im Sinne der Erfindung ist die Schaffung und Aufrechterhaltung von Bedingungen, die ein Wachstum von (ausgewählten) Organismen gewährleisten. Damit kann auch deren Vermehrung verbunden sein. Kultiviert werden unterschiedliche Organismen unter unterschiedlichen Bedingungen. Die vorliegende Erfindung bezieht sich auf die Kultivierung von Mikroorganismen in festen oder flüssigen erfindungsgemäßen Nährmedien.

Als "Probe" im Sinne der Erfindung wird eine Vorrichtung verstanden, die Mikroorganismen enthält und diese steril und vor äußeren Einflüssen geschützt aufbewahrt. Die Probe selbst kann von Oberflächen entnommen werden, ohne dass diese beschädigt werden, z.B. in Form einer Wischprobe. Es ist allerdings auch möglich, dass Teile eines festen, flüssigen oder körnigen Objekts oder auch das ganze Objekt als Probe verwendet werden. Derartige Proben können z.B. Teile von Rohstoffen oder Zwischen- und Endprodukten der Lebensmittel verarbeitenden Industrie sein, wie etwa Proben aus Flüssigzucker (flüssige Probe), Marzipan (feste Probe) oder Kakaopulver (körnige/granulatartige Probe).

In verschiedenen Ausführungsformen des erfindungsgemäßen Verfahrens kann sich im Anschluss an die Isolierung und/oder Anreicherung der osmotoleranten Mikroorganismen nachfolgend ein mikroskopisches, biochemisches, immunologisches, massenspektrometrisches oder molekularbiologisches Identifizierungsverfahren anschließen. Vorzugsweise können diese Verfahren Fluoreszenz *in situ* Hybridisierung (FISH), (real-Time) PCR-Verfahren, isothermale DNA-Amplifizierung, Enzym-Immunoassays (z.B. Enzyme Linked Immunosorbent Assay), Matrix-unterstützte Laser-Desorption/Ionisation und Massenspektrometrie mit Flugzeitanalysator (MALDI-TOF MS) oder Impedanzmessung sein.

In verschiedenen Ausführungsformen der Erfindung ist der osmotolerante Mikroorganismus eine Hefe, vorzugsweise eine Hefe ausgewählt aus der Gattung *Zygosaccharomyces, Schizosaccharomyces, Torulaspora, Candida, Wickerhamomyces, Pichia, Starmerella* und *Debarymyces.*

Der Begriff "Hefe", wie hierin verwendet, bezieht sich auf einzellige Pilze, die sich durch Sprossung oder Teilung (Spaltung) vermehren, weshalb sie synonym auch als Sprosspilze bezeichnet werden. Die meisten gehören der Abteilung der Schlauchpilze (Ascomycota) an. Es werden aber auch Entwicklungsstadien anderer Pilze als Hefen bezeichnet. Beispiele für Ständerpilz-Hefen (Basidiomycota) sind die Sprossstadien der verschiedenen Nacktbasidien-Arten (Exobasidium), bestimmte Entwicklungsstadien vieler Brandpilze oder sogar fakultativ humanpathogene Pilze wie *Malassezia furfur.* Hefen vermehren sich asexuell durch Sprossung oder Teilung. Auch sexuelle Fortpflanzung durch Sporenbildung kommt vor. Als Eukaryoten sind Hefen im Allgemeinen wesentlich größer als die weitaus meisten Bakterien und besitzen typische Zellstrukturen der Eukaryoten: komplexe Membranstrukturen, Chromosomen und eine Vielzahl von Organellen einschließlich der Mitochondrien und des endoplasmatischen Retikulums, Strukturen, die bei Prokaryoten (Bakterien und Archaeen) nicht vorhanden sind. Etwa 700 Hefearten sind heute mit über 5.000 Stämmen bekannt. Die meisten Hefen besitzen die Fähigkeit zur alkoholischen Gärung und zur Vermehrung durch Zellteilung.

In verschiedenen Ausführungsformen der Erfindung ist die Probe entnommen oder ausgewählt aus Nahrungsmitteln, vorzugsweise Flüssigzucker, Marzipan, Persipan, Schokoladenprodukt, Nougat, Honig, Sirup (z. B. Invertzuckersirup, Glucosesirup, Fructosesirup, Melasse), Zuckerdicksaft, Fruchtkonzentrat, Konfitüre, Limonade, Fleischprodukt, Feinkostsalate, Kondensmilch, Trockenfrüchte oder Sojasauce, Futtermitteln, pharmazeutischen Produkten, Kosmetika und klinisch-diagnostischen Proben.

"Nahrungsmittel", wie hierin verwendet, bezeichnet Lebensmittel, die vorwiegend der Ernährung des Menschen dienen, Makronährstoffe (Proteine, Kohlenhydrate und Lipide) enthalten und somit dem Menschen Energie zuführen. Nahrungsmittel für andere Lebewesen werden als Futtermittel bezeichnet. "Futtermittel", wie hierin verwendet, ist ein Sammelbegriff für alle Formen von Tiernahrung. Der Begriff umfasst die Ernährungsmittel für alle von Menschen gehaltenen Tiere, wie landwirtschaftliche Nutztiere, Sport- oder Heimtiere. Der Begriff "Flüssigzucker", wie hierin verwendet, bezeichnet wässerige Lösungen, die aus Glucose, Fructose, Saccharose oder Invertzuckersirup (= Saccharose + Glucose und Fructose) und Wasser bestehen und immer klare und helle Sirupe sind. Gegenüber Kristallzucker haben Flüssigzucker den Vorteil, dass sie schon gelöst und in der Regel steril filtriert und pasteurisiert sind. "Marzipan", wie hierin verwendet, ist eine Süßware aus gemahlenen Mandeln, Zucker und - je nach Herkunft - beigefügten Aromastoffen. Die aus blanchierten und geschälten Mandeln und Zucker oder Honig hergestellte Masse wird als Marzipanrohmasse bezeichnet. Im Gegensatz dazu bezeichnet "Persipan" eine dem Marzipan ähnliche Süßware, die aus Pfirsich- oder Aprikosenkernen hergestellt wird, und ein wenig herb-bitter schmeckt. Der Name ist ein Kunstwort aus lateinisch "persicus" (Pfirsich) und "Marzipan". "Schokoladenprodukt" oder "Schokolade" ist ein Lebens- und Genussmittel, dessen wesentliche Bestandteile Kakaoerzeugnisse und Zuckerarten, im Falle von Milchschokolade auch Milcherzeugnisse sind. Schokolade wird in reiner Form genossen und als Halbfertigprodukt weiterverarbeitet. Im Vergleich dazu bezieht sich der Begriff "Nougat" auf verschiedene Konfektmassen bzw. hieraus gefertigte Süßwaren, wobei grundsätzlich zu unterscheiden ist zwischen dunklem Nougat mit Kakao und weißem Nougat ohne Kakao. Die Hauptzutat von dunklem Nougat sind Haselnüsse. Die Kerne werden bei 150-180 °C etwa 20 Minuten lang geröstet und anschließend abgekühlt und in einer Mühle zerrieben. Dadurch entsteht eine Rohmasse ähnlich dem Marzipan, der Lecithin zugesetzt wird, um sie besser zu binden. Der Paste kann Nusskrokant beigemischt werden. Die Haselnussmasse wird mit Puderzucker, Kakaobutter, Sojafett, Kuvertüre, Vanillin und Milchpulver verknetet, und gewalzt, bis sie eine homogene Masse bildet. Wenn diese kühler wird, erstarrt sie und das Nougat kann plattiert und geschnitten werden. Weißer Nougat besteht aus einer schaumig aufgeschlagenen hellen Masse aus Saccharose, Glucosesirup, Eischnee, Gelatine, Honig sowie weiteren Zutaten wie Samenkernen (Mandeln, Nüssen oder Pistazien) und/oder kandierten Früchten. "Honig" ist ein von Honigbienen und bestimmten Ameisenarten zur eigenen Nahrungsvorsorge erzeugtes und vom Menschen genutztes Lebensmittel aus dem Nektar von Blüten oder den zuckerhaltigen Ausscheidungsprodukten verschiedener Insekten. Der Begriff "Sirup", wie hierin verwendet, bezeichnet eine dickflüssige, konzentrierte Lösung (Konzentrat), die durch Kochen und andere Techniken aus zuckerhaltigen Flüssigkeiten wie Zuckerwasser, Zuckerrübensaft, Fruchtsäften oder Pflanzenextrakten gewonnen wird. "Fruchtkonzentrat" oder "Fruchtsaftkonzentrat" bezieht sich auf eine Lösung, in der der Fruchtsaft durch physikalischen Entzug von Wasser konzentriert wird. "Limonade", wie hierin verwendet, ist ein alkoholfreies, gesüßtes und meist mit Kohlensäure versetztes Erfrischungsgetränk mit Fruchtauszügen auf Basis von Wasser. Der Begriff "Fleischprodukt" oder "Fleisch" bezeichnet im Allgemeinen Weichteile von Menschen und Tieren. Im Besonderen steht der Begriff für Teile von Säugetieren und Vögeln, die zur Ernährung des Menschen genutzt werden. Dazu zählen neben Muskelgewebe mit Fett- und Bindegewebe auch Sehnen sowie bestimmte innere Organe. Fleisch wird auch zur Bezeichnung für die als Nahrungsmittel genutzten Weichteile wechselwarmer Tiere wie Fische, Krebse, Muscheln und Schnecken verwendet. "Kondensmilch", wie hierin verwendet, wird aus Milch unter partiellem Wasserentzug hergestellt. Durch erneute Zugabe von Wasser kann so wieder ein H-Milchähnliches Produkt erhalten werden. Der Begriff "Trockenfrüchte" oder "Trockenobst" bezieht sich auf gedörrtes Obst mit einer Restfeuchtigkeit von etwa 20 %. "Sojasauce", wie hierin verwendet, ist eine asiatische Würzsauce, die aus Wasser, Sojabohnen, Salz und (regional begrenzt) Getreide hergestellt wird und sich zum Würzen und Verfeinern von Speisen eignet. Der Begriff "pharmazeutische Produkte", wie hierin verwendet, wird synonym zu Arzneimittel, Fertigarzneimittel oder auch für Zubereitungsformen von Arzneistoffen verwendet. Eine nicht einschränkende Liste an Beispielen für arzneiliche Zubereitungen sind Tabletten, Dragees, Salben, Cremes, Lotionen, Tinkturen und Infusionslösungen. "Kosmetika" sind Stoffe oder Gemische, die dazu bestimmt sind, äußerlich am Körper des Menschen oder in seiner Mundhöhle angewendet zu werden. Ihre ausschließliche oder überwiegende Aufgabe ist es, Haut, Haare, Nägel, Lippen, die äußeren Intimregionen oder die Zähne und die Mundschleimhäute zu reinigen, zu schützen, in gutem Zustand zu erhalten, zu parfümieren, deren Aussehen zu verändern oder den Körpergeruch zu beeinflussen. Eine "klinischdiagnostische Probe" im Sinne der Erfindung ist ein infektiöses Material, das Krankheitserreger enthält oder enthalten kann. Die Proben werden in die UN-Gefahrenklasse 6.2 eingestuft und bestehen u.a., nicht einschränkend, aus Ausscheidungsstoffen, Sekreten, Blut oder Blutbestandteilen, Geweben oder Gewebeflüssigkeiten von Mensch und Tier, die zu Untersuchungs- und Forschungszwecken befördert werden.

Ein vierter Aspekt der Erfindung ist gerichtet auf ein Kit zur Untersuchung von osmotoleranten Mikroorganismen, umfassend: das erfindungsgemäße Nährmedium, ein Kulturgefäß und eine Vorrichtung zum Nachweis der Gasbildung der osmotoleranten Mikroorganismen.

Der Begriff "Kit", wie hierin verwendet, bezieht sich auf verpackte Reagenzien zur Untersuchung von osmotoleranten Mikroorganismen. Dementsprechend umfasst ein solches erfindungsgemäßes Kit die oben beschriebenen Komponenten. Unter einem Kulturgefäß im Sinne der Erfindung kann, vorzugsweise, eine Flasche mit Verschluss, ein Reagenzglas, Erlenmeyerkolben, Schraubdeckelgefäß jeweils aus den Materialien Glas oder Kunststoff verstanden werden. Die Vorrichtung zum Nachweis der Gasbildung der osmotoleranten Mikroorganismen ist in bestimmten Ausführungsformen der Erfindung ein Durhamröhrchen. Dieses wird so in das in dem Kulturgefäß befindliche erfindungsgemäße Nährmedium eingebracht, dass es bei aufrechter Lage völlig mit dem Nährmedium gefüllt ist. Das Nährmedium wird mit Mikroorganismen eingeimpft, deren Gasbildung bei der Gärung im Durhamröhrchen gesammelt wird und zu erkennen ist. Die Vorrichtung zum Nachweis der Gasbildung der osmotoleranten Mikroorganismen ist in bestimmten Ausführungsformen der Erfindung eine Septumflasche mit Einwegspritze und Kanüle. Dabei ragt die Kanüle in den Gasraum des Kulturgefäßes. Die Gasbildung wird durch die Bewegung des Spritzenkolbens anzeigt. Die Vorrichtung zum Nachweis der Gasbildung der osmotoleranten Mikroorganismen ist in bestimmten Ausführungsformen der Erfindung ein pH-Wert-Indikator, Druck- oder CO₂-Sensor oder eine Farbreaktion, die durch eine biochemische Reaktion in Verbindung mit einem Farbindikator hervorgerufen wird. Zusätzlich kann das Kit auch eine Beschreibung zur Handhabung der beschriebenen Komponenten oder auch Informationen über dem Fachmann bekannte weitere verwendbare Reagenzien enthalten.

### BEISPIELE

### Material und Methoden

Das Wachstum und Gasbildungsvermögen osmotoleranter Hefen wurde in ausgewählten Nährmedien im Parallelfermenter überprüft. Alle Nährmedien wurden wie in Tabelle 1 beschrieben, eingewogen und autoklaviert. Bei den hoch zuckerhaltigen Medien erfolgte die Zuckerzugabe unter ständigem Rühren, damit ein vollständiges Lösen der Bestandteile möglich war. Grundsätzlich wurden nach jeder Medienherstellung jeweils die a_{w}-Werte überprüft und ggf. die pH-Werte eingestellt. Die Messung der a_{w}-Werte erfolgte mit dem Aqua Lab 4TE DUO (Decagon Devices Inc., Pullman, USA). Begleitend zu den Wachstumsversuchen im Parallelfermenter wurden Keimzahlbestimmungen auf entsprechenden Kulturmedien, verfestigt mit 1,5 % Agar, durchgeführt.

**Tabelle 1: Nährmedien zum Nachweis osmotoleranter Hefen**

| **Medium** | **Zusammensetzung** | **Konzentration** | **a_{w}-Wert** | **pH-Wert** |
|---|---|---|---|---|
| MYG 50 | Glucosemonohydrat | 500 g | 0,89 | 5,6 |
| | Malzextrakt | 10 g | | |
| | Hefeextrakt | 2,5 g | | |
| | dest. Wasser | 500 mL | | |
| GB 50 | Glucosemonohydrat | 500 g | 0,91 | 4,5 |
| | Hefeextrakt | 5 g | | |
| | dest. Wasser | 495 g | | |
| DG 18 | Glucosemonohydrat | 10 g | 0,95 | 5,6 |
| | Casein-Pepton | 5 g | | |
| | Dichloran | 0,002 g | | |
| | Chloramphenicol | 0,1 g | | |
| | KH₂PO₄ | 1 g | | |
| | MgSO₄ · H₂O | 0,5 g | | |
| | Glycerin | 220 g | | |
| | dest. Wasser | 1000 mL | | |

### Organismen

Im Rahmen der Untersuchungen wurden die in Tabelle 2 angegebenen Kultur- und Wildstämme eingesetzt. Es wurden gezielt Hefestämme von Kulturensammlungen bezogen, die ursprünglich aus hoch zuckerhaltigen Rohstoffen und Produkten stammen. Zusätzlich wurden Wildstämme aus ausgewählten Matrizes isoliert. Die Identifizierung der Wildstämme erfolgte durch Sequenzierung ihrer Internal Transcibed Spacer (ITS)-Regionen. Alle Identifizierungsergebnisse wurden darüber hinaus durch Identifizierung mittels MALDI-TOF-MS bestätigt.

Vor jedem Wachstumsversuch wurde eine Impföse des gewünschten Hefestammes auf Schrägagar in 5 ml des zu untersuchenden Nährmediums gegeben und für 3-5 Tage bei 30 °C inkubiert. Die Vorkulturen, die eine Keimzahl von 10⁷ bis 10⁸ KbE/mL enthielten, wurden in dekadischen Reihen in Reagenzgläsern mit 9 mL des zu untersuchenden Mediums verdünnt und zur Inokulation der Versuchsansätze verwendet.

**Tabelle 2: Untersuchte osmotolerante Hefen (Kultur- und Wildstämme)**

| **Stamm** | **Gattung, Art** | **Isoliert aus** |
|---|---|---|
| DSMZ 70492 | *Zygosaccharomyces bailii* | Apfelsaft |
| DSMZ 70526 | *Torulaspora delbrueckii* | Marzipan |
| DSMZ 70576 | *Schizosaccharomyces pombe* | Traubenmost |
| CBS 1091 | *Zygosaccharomyces mellis* | Honig |
| CBS 4512 | *Zygosaccharomyces rouxii* | Marzipan |
| WH 1002 | *Zygosaccharomyces rouxii* | Marzipan |
| WH 1004 | *Zygosaccharomyces rouxii* | Zucker |
| WH 1005 | *Zygosaccharomyces rouxii* | Dattel |
| WH 1010 | *Candida parapsilosis* | Schokoladentrüffel |

| | | |
|---|---|---|
| DSMZ: Deutsche Sammlung von Mikroorganismen und Zellkulturen, Braunschweig; CBS: Centraalbureau voor Schimmelcultures, Utrecht, Holland; WH: Wildhefe | | |

### Untersuchung der Gasbildung im Parallelfermenter

Zur Ermittlung der Stoffwechselaktivität in den beschriebenen Kulturmedien wurden Vergleichsuntersuchungen unter standardisierten Kultivierungsbedingungen im RAMOS®-System (HiTec Zang GmbH, Herzogenrath) durchgeführt. Das Gerät erlaubt die parallele Untersuchung von acht Fermentationsansätzen unter quantitativer Messung der CO₂-Bildung. Temperatur und Rotation des Schüttlers sind regulierbar. Die CO₂-Messung erfolgt im RAMOS®-System in speziellen Messkolben indirekt über Sauerstoff- und Drucksensoren (Anderlei, T. et al. (2004), Biochem Eng J 17, 187-194). Die RAMOS®-Software zeigt unter anderem Messgrößen wie die Kohlendioxidtransferrate (CTR), Kohlendioxidtransfer (CT), Sauerstoffpartialdruck und Differenzdruck. Die Messdaten können sowohl für jeden Fermentationskolben einzeln als auch im direkten Vergleich aller acht Fermentationskolben ermittelt werden. Die ausgewählten Hefestämme (Tab. 2) wurden in den Kulturmedien GB 50, MYG 50, DG 18 und OM (erfindungsgemäßes Nährmedium aus Beispiel 1) im Parallelfermenter untersucht. Die Messungen im Parallelfermenter erfolgten in speziellen 250-ml-Kulturkolben, die mit je 25 mL Nährmedium befüllt waren. Die Versuche im Parallelfermenter wurden bei einer Bebrütungstemperatur von 30 °C unter Schütteln (60 UpM) durchgeführt. Inokuliert wurden 10 bzw. 100-1000 KbE/mL der jeweiligen Hefereinkultur. Die Untersuchung von Wachstum und Gasbildung erfolgte über einen Zeitraum von max. 10 Tagen. Die Messzeit des Systems wurde nach Vorgaben des Herstellers auf 10 min eingestellt, die Spülzeit der Kolben betrug 20 min, sodass sich Messzyklen von 30 min ergaben. Da die Hefen ohne Sauerstoffzufuhr inkubiert werden sollten, wurde die Durchflussspülphase des Systems (Aktivität der Pumpe) auf einen Fluss von 0 mL/min eingestellt. Unter diesen Bedingungen war der vorhandene Luftsauerstoff in den Fermentationskolben durch das Wachstum der Hefen nach einiger Zeit vollständig verbraucht. Vor Beginn jeder Messung im RAMOS®-System wurden jeweils eine Sauerstoffkalibrierung und ein Dichtigkeitstest durchgeführt. Um die Messkurven vergleichen zu können, wurden für die Auswertung der Gäraktivität charakteristische Zeitpunkte definiert. Es wurden die Zeitpunkte der CO₂-Transferrate > 0,004 mol/L x h herangezogen, die den Beginn der Fermentation zeigen. Außerdem wurden Zeitpunkte und Höhe der maximalen Gäraktivität (CTR_{max.}) untereinander verglichen.

Begleitend zu den Versuchen im Parallelfermenter erfolgte die Keimzahlbestimmung auf den zu untersuchenden Nährmedien, verfestigt mit 1,5 % Agar. So wurden zu Beginn und am Ende jedes Versuches Proben für die Keimzahlbestimmungen aus den Fermentationskolben entnommen.

### Beispiel 1: Zusammensetzung eines Basis-Nährmediums

Die Entwicklung eines Nährmediums, das bei hoher Selektivität für osmotolerante Hefen, d.h. a_{w}-Werten um 0,90, ein schnelleres Wachstum ermöglicht als die bekannten Medien GB 50 und MYG 50 wurde anvisiert. Die Absenkung der a_{w}-Werte wird in den Nährmedien GB 50 und MYG 50 wird ausschließlich durch einen Gehalt von 50 Gew.-% Glucose erreicht. Dazu wurden Nährmedien mit Mischungen aus Glucose, Fructose und Hefeextrakt hergestellt und Wachstumsversuche mit *Z*. *rouxii* (WH 1002) im BioLumix™-System (I&L Biosystems GmbH, Königswinter) durchgeführt (Tab. 3). Parallel wurden Nährmedien mit unterschiedlichen Konzentrationen an Glucose, KCl, Glycerin und Hefeextrakt hergestellt und Wachstumsversuche im BioLumix™-System durchgeführt (Tab. 4).

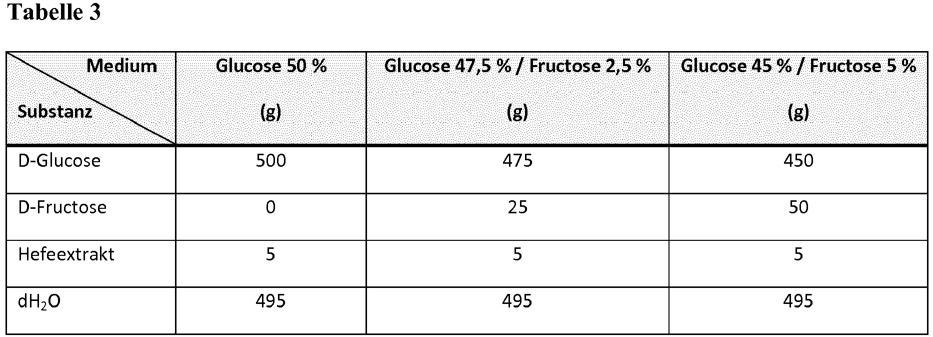

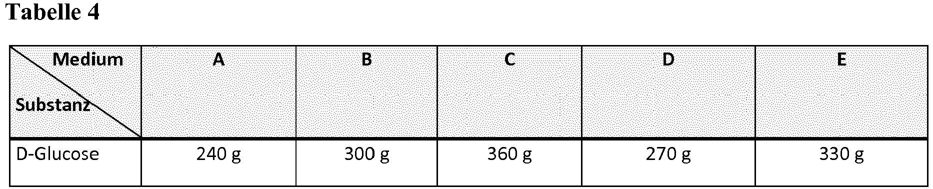

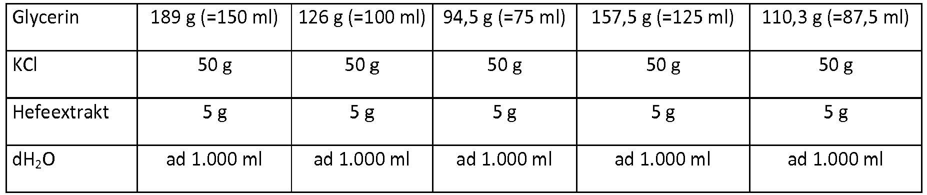

Ein Nährmedium aus 45 Gew.-% Glucose, 5 Gew.-% Fructose, 0,5 Gew.-% Hefeextrakt und 49,5 Gew.-% Wasser zeigte das schnellste Wachstum von *Z*. *rouxii* (WH 1002) im Vergleich zum Wachstum in den Nährmedien aus 50 Gew.-% Glucose, 0,5 Gew.-% Hefeextrakt und 49,5 Gew.-% Wasser und 45 Gew.-% Glucose, 5 Gew.-% Fructose, 0,5 Gew.-% Hefeextrakt und 49,5 Gew.-% Wasser (Abb. 1A).

Durch Kombination der Substanzen D-Glucose, Fructose, KCl, Glycerin und Hefeextrakt in den o.a. Konzentrationen war es möglich, ein Nährmedium herzustellen, das bei gleichen a_{w}-Werten (0,90 ± 0,1) wie die Nährmedien des Standes der Technik (GB 50, MYG 50) zu schnellerem Hefewachstum führte.

Die Untersuchungen zum Einfluss der Medienzusammensetzung aus Glucose, KCl und Glycerin zeigten, dass 30 Gew.-% Glucose, 12,6 Gew.-% Glycerin, 5 Gew.-% KCl, und 0,5 Gew.-% Hefeextrakt die besten Resultate in Bezug auf schnelles Wachstum lieferte. So war das Wachstum von *Z*. *rouxii* (WH 1002) bei der Untersuchung im BioLumix™-System schneller, als im Nährmedium MYG 50, das einen vergleichbaren a_{w}-Wert besitzt (Abb. 1B).

### Beispiel 2: Zugabe einzelner Substanzen zu einem Grundmedium zur Steigerung des Gärstoffwechsels osmotoleranter Hefen

Es sollte durch das Nährmedium der Gärstoffwechsel osmotoleranter Hefen gesteigert werden, um das primäre Verderbspotential der Hefen (d.h. die Gasbildung in Produkten) sensitiver und dadurch schneller erfassen zu können.

Es wurden Medienzusätze in verschiedenen Konzentrationen zu einem Grundmedium aus 50 Gew.-% Glucose und 0,5 Gew.-% Hefeextrakt gegeben. Dieses Nährmedium entspricht in seiner Zusammensetzung dem Nährmedium GB 50 (Jermini, M. et al. (1987), J. Food Prot. (SO), 468-472). Die CO₂-Transferrate (CTR) osmotoleranter Hefen wurde im RAMOS®-Parallelfermenter im Vergleich zum Grundmedium untersucht.

Vor allem Malzextrakt (10 g / l), Pepton (5 g / l) und Ammoniumsalze (NH₄NO₃ und (NH₄)₂HPO₄) führten zu einer Steigerung der Gäraktivität. Schnelleres Wachstum konnte durch Zusatz von Thiamin (Vitamin Bi) erzielt werden (Tab. 5). Weitere, in der Literatur beschriebene Substanzen wie Trehalose, Inositol, Harnstoff, ZnZl₂, L-Prolin, Glutathion wurden in verschiedenen Konzentrationen untersucht, zeigten aber keine signifikante Steigerung des Gärstoffwechsels.

**Tabelle 5**

| **Zusatz** | **Konzentration** | **Zeit bis CTR_{max.} (h)** | | **Zeit-differenz (h)** | **CTR_{max.} (mol / L** · **h)** | | **Differenz** |
|---|---|---|---|---|---|---|---|
| | | **Grund-medium** | **+ Zusatz** | | **Grund-medium** | **+ Zusatz** | |
| Thiamin | 1,3 mg / l | 49 | 47,5 | -1,5 | 0,0285 | 0,0269 | -0,0016 |
| Malzextrakt | 10 g / l | 47 | 49 | + 2,0 | 0,0223 | 0,0314 | 0,0091 |
| Fleisch-Pepton | 5 g / l | 49 | 52,5 | + 3,5 | 0,0267 | 0,0324 | 0,0057 |
| Casein-Pepton | 5 g / l | 49 | 52 | + 3,0 | 0,0267 | 0,0306 | 0,0039 |
| NH₄NO₃ | 2 g / l | 48 | 52,5 | + 4,5 | 0,0243 | 0,0269 | 0,0026 |
| (NH₄)₂HPO₄ | 2 g / l | 48 | 52,5 | + 4,5 | 0,0243 | 0,0279 | 0,0036 |
| MgSO₄ | 1 g / l | 49 | 49 | ± 0 | 0,0285 | 0,0282 | -0,0003 |

### Beispiel 3: Zusammenstellung von Nährmedien

Anvisiert wurde die Herstellung von Nährmedien, die im Vergleich zu den Nährmedien zu einem schnelleren Wachstum und einer erhöhten Gäraktivität führen. Nach den Untersuchungen zur Absenkung der a_{w}-Werte und den Untersuchungen einzelner Nährmedienzusätze zur Förderung der Gäraktivität im RAMOS®-Parallelfermenter wurden neue Nährmedien auf Basis der Untersuchungsergebnisse zusammengestellt. Es wurde ein Nährmedium bestehend aus 21,92 Gew.-% Glucose, 1,15 Gew.-% Fructose, 0,38 Gew.-% Hefeextrakt, 9,69 Gew.-% Glycerin, 3,85 Gew.-% KCl, 0,08 Gew.-% MgSO₄, 0,15 Gew.-%

(NH₄)₂HPO₄, 0,0001 Gew.-% Thiamin und 62,78 Gew.-% Wasser (hier als Medium A bezeichnet) und ein Nährmedium bestehend aus 21,92 Gew.-% Glucose, 1,15 Gew.-% Fructose, 0,38 Gew.-% Hefeextrakt, 0,38 Gew.-% Pepton, 9,69 Gew.-% Glycerin, 3,85 Gew.-% KCl, 0,08 Gew.-% MgSO₄, 0,15 Gew.-% (NH₄)₂HPO₄, 0,0001 Gew.-% Thiamin und 62,40 Gew.-% Wasser (hier als Medium B bezeichnet) und ein Nährmedium bestehend aus 21,92 Gew.-% Glucose, 1,15 Gew.-% Fructose, 0,38 Gew.-% Hefeextrakt, 0,77 Gew.-% Malzextrakt, 0,38 Gew.-% Pepton, 9,69 Gew.-% Glycerin, 3,85 Gew.-% KCl, 0,08 Gew.-% MgSO₄, 0,15 Gew.-% (NH₄)₂HPO₄, 0,08 Gew.-% (NH₄)₂SO₄, 0,0001 Gew.-% Thiamin und 61,54 Gew.-% Wasser (hier als Nährmedium "OM" bezeichnet) hergestellt.

Die folgenden Werte wurden für die maximale CO₂-Transferrate (CTR_{max.}) von *Z*. *rouxii* (WH 1001) ermittelt: GB 50: 0,0175 mol · 1 · h, Medium A: 0,0308 mol · 1 · h, Medium B: 0,0365 mol · 1 · h, OM: 0,0516 mol · 1 · h (Abb. 2 und Abb. 3). Damit führte die Addition ausgewählter Substanzen zu einer stufenweisen Anhebung der maximalen CO₂-Transferrate.

Im Ergebnis führt die Zugabe einer Kombination von ausgewählten Substanzen, die bereits einzeln positive Effekte auf den Gärstoffwechsel osmotoleranter Hefen zeigten, zu einer erheblichen Steigerung des Gärstoffwechsels.

### Beispiel 4: Herstellung eines erfindungsgemäßen Nährmediums

Ein erfindungsgemäßes Nährmedium mit den nachstehend aufgelisteten Komponenten wurde wie unten beschrieben hergestellt (im Folgenden mit OM abgekürzt):
285 g D(+)-Glucosemonohydrat (Merck KGaA)
15 g D(-)-Fructose (Merck KGaA)
5 g Hefeextrakt (Merck KGaA)
10 g Malzextrakt (Merck KGaA)
5 g Pepton aus Fleisch LP0037 (Oxoid GmbH)
100 ml Glycerin (99,5 %, p.a.) (Carl Roth GmbH)
50 g Kaliumchlorid (KCl) (Merck KGaA)
1g Magnesiumsulfat (MgSO₄ • 7H₂O) (Merck KGaA)
2 g Diammoniumhydrogenphosphat ((NH₄)₂HPO₄) (Merck KGaA)
1g Diammoniumsulfat ((NH₄)₂SO₄) (Merck KGaA)
1,3 mg Thiaminchloridhydrochlorid (Merck KGaA)
800 ml entmineralisiertes Wasser (VE-Wasser)

Zur Medienherstellung wird die angegebene Menge Zucker (Glucosemonohydrat und Fructose) eingewogen und mit 300 ml VE-Wasser gelöst und für 10 Minuten bei 121 °C autoklaviert. Die übrigen Substanzen (außer Thiaminchloridhydrochlorid) werden mit 500 ml VE-Wasser gelöst und unter den gleichen Bedingungen separat autoklaviert. Nach dem Autoklavieren werden beide Lösungen unter sterilen Bedingungen zusammengeführt. Thiaminchloridhydrochlorid wird in 2 ml VE-Wasser gelöst, mit Hilfe einer Einwegspritze mit PES-Spritzenvorsatzfilter (Porendurchmesser: 0,2 µm; Pall Corp., Newquay, U.K.) sterilfiltriert und dem abgekühlten Nährmedium zugegeben. Das so hergestellte Nährmedium besitzt einen pH-Wert von 5,7 und einen a_{w}-Wert von 0,90. Dieses Nährmedium wird in den folgenden Beispielen als "OM" bezeichnet.

### Beispiel 5: Vergleich der CO₂-Transferrate osmotoleranter Hefen in GB 50 und OM

In den Beispielen wird die Stoffwechselaktivität osmotoleranter Hefen der Arten *Zygosaccharomyces rouxii* (WH 1001, WH 1002, WH 1004, WH 100S, CBS 4512), *Zygosaccharomyces bailii* (DSMZ 70492), *Zygosaccharomyces mellis* (CBS 1091), *Torulaspora delbrueckii* (WH 1013, DSMZ 70526), *Candida parapsilosis* und *Schizosaccharomyces pombe* (DSM 70576) in OM mit der Stoffwechselaktivität in Nährmedien des Standes der Technik: GB 50 (Jermini et al. (1987) J. Food Prot. (SO), 468-472), MYG 50 (Beuchat (1993) International Journal of Food Microbiology 19 (1), 1-14) und DG 18 (Hocking und Pitt (1980) Appl Environ Microbiol 39 (3), 488-492) vergleichen. Die Nährmedien wurden mit einem Ausgangstiter von jeweils 10¹-10² KbE/ml inokuliert. Der Nährmedienvergleich erfolgte im RAMOS®-Parallelfermenter (HiTec Zang GmbH, Herzogenrath), der die Untersuchung von Fermentationsansätzen (25 ml Nährmedium in 250 ml RAMOS®-Fermentationskolben) unter quantitativer CO₂-Messung erlaubt. Abbildung 4 zeigt das Ergebnis des direkten Vergleichs der CO₂-Transferrate (CTR) osmotoleranter Hefen beim Wachstum in GB 50 und OM im Parallelfermenter.

Bei der Hefe Z. *rouxii* (WH 1001) wurde der Wert CTR > 0,004 mol / 1·h, der den Beginn der Gärung zeigt, in OM um 2 h früher erreicht, als in GB 50. Die maximale Gäraktivität (CTR_{max.}) war in OM um das 2,9-fache höher als in GB 50 (Abb. 4A). Bei *T. delbrueckii* (WH 1013) setzte die Gäraktivität (CTR > 0,004 mol / 1·h) um 25 h früher ein, als in GB 50. Ähnlich wie bei Z. *rouxii* war die maximale Gäraktivität (CTR_{max.}) mit einem Wert von 0,0389 mol / 1·h um das 2,9-fache höher als in GB 50 (Abb. 4B). Die CTR von *S. pombe* (DSM 70576) in OM zeigte ein um 11 h schnelleres Einsetzen der Gärung und eine um Faktor 2,3 gesteigerte CTR_{max.} im Vergleich zum Wachstum in GB 50 (Abb. 4C).

### Beispiel 6: Vergleich von Nachweiszeiten bis zum Einsetzen der Gärung in GB 50 und OM

Die CO₂-Transferraten weiterer osmotoleranter Hefen, kultiviert in den Nährmedien GB 50 und OM wurden ermittelt. Bei allen untersuchten Hefen wurde in OM ein schnelleres Einsetzen der Gärung festgestellt (Abb. 5). Der größte Zeitgewinn wurde beim Wachstum von *T. delbrueckii* (DSM 70526) in OM erzielt, bei der die Gärung 47 h früher als in GB 50 einsetzte.

### Beispiel 7: Vergleich der maximalen Gäraktivität (CTR_{max.}) osmotoleranter Hefen in verschiedenen Nährmedien

In Hinblick auf die maximale CO₂-Transferrate (CTR_{max.}) wurden die Nährmedien GB 50 (Jermini et al. (1987) J. Food Prot. (SO), 468-472), MYG 50 (Beuchat (1993) International Journal of Food Microbiology 19 (1), 1-14) und DG 18 (Hocking und Pitt (1980) Appl Environ Microbiol 39 (3), 488-492) mit OM im RAMOS®-Parallelfermenter verglichen. Außer dem Nährmedium DG 18 des Standardverfahrens (ISO/FDIS 21527-2) besitzen alle Nährmedien a_{w}-Werte ≤ 0,91. Bei den Nährmedien GB 50 und MYG 50 handelt es sich um Medien, deren a_{w}-Werte durch die Zugabe von 50 % Glucose (w/w) auf 0,89 - 0,91 abgesenkt werden. Der Vergleich des erfindungsgemäßen Nährmediums OM mit GB 50, MYG 50 und DG 18 ergab für Z. *rouxii* eine Zeitspanne von 54 h bis zur maximalen CO₂-Transferrate in OM. Die CTR_{max.} war im OM mit 0,052 mol / 1·h um den Faktor 2,9 höher als in GB 50 (Abb. 6 A). Bei *T. delbrueckii* (WH 1013) konnte die Zeit bis CTR_{max.} im Vergleich zu GB 50 um 27,5 h verkürzt werden. Die Gäraktivität war mit 0,039 mol / 1·h um den Faktor 2,9 höher als in GB 50 (Abb. 6 B). Ähnliche Werte wurden bei der Untersuchung der Hefen Z. *mellis* und *S. pombe* ermittelt. Die CTR_{max.} war in OM um den Faktor 2,4 bzw. 2,8 höher als im Vergleich zu GB 50 (Abb. 6 C und D). Beim Vergleich des Gärstoffwechsels osmotoleranter Hefen in OM mit DG 18 wurde der Vorteil eines erfindungsgemäßen Nährmediums noch deutlicher gezeigt. Im Vergleich zu DG 18 war die maximale CO₂-Transferrate in OM um den Faktor 5,0 bis 11,7 höher (Abb. 6 A-D).

### Beispiel 8: Vergleich des OM Kulturmediums mit bekannten Medien im Parallelfermentersystem

Die respiratorischen Quotienten (CTR/OTR) wurden für jeden Gärkolben durch die RAMOS Parallelfermenter Software berechnet. Ein respiratorischer Quotient >1 zeigt an, dass der Sauerstoffverbrauch kleiner war als die Kohlendioxidproduktion. Diese Versuche zeigen, dass, selbst wenn sich zu Beginn der Experimente Sauerstoff im Gasgemisch der Fermentationskolben befand, der Hefestoffwechsel auf Fermentation basiert. Unabhängig von dem getesteten Kulturmedium erreichte die Keimzahlbestimmung Mittelwerte von 5,0·10⁷ KbE/ml am Ende der Experimente in den Fermentationskolben.

Das Wachstum der *Z*. *rouxii* Stämme war das schnellste in allen getesteten Kulturmedien im Vergleich zu den untersuchten *Zygosaccharomyces-Arten* oder den anderen Hefespezies. Dies kann dadurch erklärt werden, dass diese Art die Fähigkeit besitzt sich Umgebungen mit extrem niedrigen Wasseraktivitäten anzupassen (Fleet, 1992). Für C. *lusitaniae* (WH 1015) konnte kein Wachstum in GB 50 und MYG 50 Bouillon beobachtet werden. Innerhalb aller 23 getesteten Hefestämme variierten die Detektionszeiten von 31,5 h bis 82 h in OM Bouillon je nach Spezies und Stamm bei einer anfänglichen Keimzahl von 10² KbE/ml (Abb. 7a). Im Gegensatz dazu, variierten die Detektionszeiten in den zwei vergleichbaren Kulturmedien mit a_{w}-Werten um 0,9 zwischen 38,5 h bis 128 h (GB 50 Bouillon) und 34 h bis 95,5 h (MYG 50 Bouillon). Das Wachstum der Hefen konnte für die meisten Teststämme verbessert werden, wenn OM Bouillon anstelle von GB 50 und MYG 50 verwendet wurde, was durch verkürzte Detektionszeiten nachgewiesen wurde. Die Ergebnisse zeigen, dass Detektionszeiten von 2-3 Tagen für die Mehrheit der getesteten osmotoleranten Hefearten mit Anfangstiter von 10² KbE/g in OM Bouillon erreicht werden können.

Das weniger selektive Kulturmedium DG18 Bouillon, mit einem a_{w}-Wert von 0,95, verursachte ein schnelleres Wachstum der Hefen im Vergleich zu Kulturmedien mit a_{w}-Werten um 0,90. *Z*. *mellis* (CBS 1091) und C. *magnoliae* (WH 1023) wuchsen nicht in DG18 Bouillon. Die Fermentationsraten in DG18 waren die niedrigsten aller untersuchten Kulturmedien. Beispielsweise war der Mittelwert der CTR_{max.} für die getesteten *Zygosaccharomyces*-Spezies 5,9 -fach höher in OM Bouillon verglichen mit DG18 Bouillon und die CTR_{max.} der getesteten *S. pombe* Stämme war um den Faktor 8,9 erhöht. Insgesamt wurden erhebliche Steigerungen der CTR_{max.} mit dem entwickelten Kulturmedium (OM) erreicht (Fig. 7b).

Neben der geringeren Gesamtmenge an vergärbarer Glucose in DG18 im Vergleich zu den anderen 3 untersuchten Kulturmedien, könnte in diesem Kulturmedium die Anwesenheit von Dichloran einen weiteren negativen Einfluss auf die Hefe-Fermentation haben (Michaels et al., 1977; Henson, 1981). Daher ist dieses Kulturmedium weniger geeignet für einen selektiven qualitativen Test auf osmotolerante Hefen, obwohl das Wachstum definierter Hefekolonien auf DG18-Agar beobachtet werden konnte. Diese Aussage wird von Beuchat et al. (2001) gestützt, die zeigen, dass DG18 nicht als allgemeines Medium der Wahl zur Anzucht von Hefen aus trockenen Lebensmitteln verwendet werden kann. Abb. 8 gibt ausführlichere Informationen über die Detektionszeiten und die maximalen Gärungsraten für jeden Stamm auf dem jeweils getesteten Kulturmedium.

### Beispiel 9 : Inokulationsexperimente im Bioluminix System

Durch den Einsatz von OM Kulturmedium in Verbindung mit einem automatisierten Testinkubator sollten die erhöhten Fermentationsraten von Hefen in diesem Kulturmedium für einen empfindlichen und schnellen Nachweis genutzt werden. Für die experimentelle Inokulation stark zuckerhaltiger Lebensmittel wurden Proben mit 10¹ bis 10² KbE/g kontaminiert. Keimzahlbestimmungen der beimpften Nahrungsmittelsuspension aus den Biolumix™ Teströhrchen auf OM-Agar zeigten, dass die Anfangskeimzahl zwischen 1,0·10¹ KbE/ml und 3,5·10² KbE/ml variierte. Ohne Lebensmittelmatrix waren die Detektionszeiten für *Z*. *rouxii* (CBS 4512 und WH 1001) zwischen 34,6 bis 36,5 h. Die Art des Lebensmittels hatte bei niedrigen Hefezahlen weniger Einfluss auf die Detektion von *Z*. *rouxii,* was zu einem Mittelwert von 51,9 h führte (Abb. 9). Die Untersuchung von *T. delbrueckii* (DSMZ 70526 und WH 1013) in OM Kulturmedium ohne Lebensmittelmatrix führte zu Detektionszeiten von 35-37,7 h. Insgesamt waren die Detektionszeiten für *T. delbrueckii* in stark zuckerhaltigen Lebensmitteln variabler als die Detektionszeiten für *Z*. *rouxii* und hingen von der Lebensmittelmatrix ab. Die Zeiten variierten zwischen 33,9 h für die Detektion in Persipanrohmasse und 69,8 h zum Nachweis von 10 KbE/g in einer Mohnbackfüllung (Abb. 9).

Die Verwendung eines automatisierten Testinkubators in Kombination mit dem entwickelten OM Bouillon führte zu einem schnelleren Nachweis von *Z*. *rouxii* und *T. delbrueckii* in stark zuckerhaltigen Lebensmitteln im Vergleich zu den bekannten Nachweisverfahren mit Kulturmedien mit vergleichbaren a_{w}-Werten. In diesen Verfahren wurden 5 bis 7 Tage benötigt, um einen Nachweis osmotoleranter Hefearten mit einer geringen anfänglichen Keimzahl (z. B. 10 KbE/g oder ml) in Anreicherungskulturen mit stark zuckerhaltigen Lebensmitteln zu führen (Jermini et al, 1987; Beuchat, 1993). Generell ist ein Vorteil der Kombination aus einem automatisierten Testinkubator und einem Kulturmedium, das zeitaufwendige Untersuchungen auf das Vorhandensein oder die Abwesenheit der Mikroorganismen über einen Zeitraum von mehreren Tagen entfallen.

## Patentansprüche

1. Nährmedium, umfassend:
15 bis 30 Gew.-% Glucose, vorzugsweise 20 bis 25 Gew.-% Glucose,
0,7 bis 2,0 Gew.-% Fructose, vorzugsweise 1,0 bis 1,3 Gew.-% Fructose,
0,2 bis 0,8 Gew.-% Hefeextrakt, vorzugsweise 0,3 bis 0,5 Gew.-% Hefeextrakt,
0,4 bis 1,5 Gew.-% Malzextrakt, vorzugsweise 0,6 bis 1,0 Gew.-% Malzextrakt,
0,2 bis 0,8 Gew.-% Pepton, vorzugsweise 0,3 bis 0,6 Gew.-% Pepton,
5 bis 15 Gew.-% Glycerin, vorzugsweise 8 bis 12 Gew.-% Glycerin,
2 bis 10 Gew.-% KCl, vorzugsweise 3 bis 5 Gew.-% KCl,
0,04 bis 0,16 Gew.-% MgSO₄, vorzugsweise 0,05 bis 0,1 Gew.-% MgSO₄,
0,04 bis 0,32 Gew.-% (NH₄)₂HPO₄, vorzugsweise 0,1 bis 0,2 Gew.-% (NH₄)₂HPO₄,
0,04 bis 0,16 Gew.-% (NH₄)₂SO₄, vorzugsweise 0,05 bis 0,1 Gew.-% (NH₄)₂SO₄,
0,00003 bis 0,0002 Gew.-% Thiamin, vorzugsweise und 0,00005 bis 0,00015 Gew.-% Thiamin, und
42,2 bis 76,3 Gew.-% Wasser, vorzugsweise 57 bis 67 Gew.-% Wasser.

2. Nährmedium nach Anspruch 1, bestehend aus:
21,92 Gew.-% Glucose,
1,15 Gew.-% Fructose,
0,38 Gew.-% Hefeextrakt,
0,77 Gew.-% Malzextrakt,
0,38 Gew.-% Pepton,
9,69 Gew.-% Glycerin,
3,85 Gew.-% KCl,
0,08 Gew.-% MgSO₄,
0,15 Gew.-% (NH₄)₂HPO₄,
0,08 Gew.-% (NH₄)₂SO₄,
0,0001 Gew.-% Thiamin und
61,54 Gew.-% Wasser.

3. Nährmedium nach einem der Ansprüche 1 oder 2, wobei
a) der pH-Wert zwischen 4,5 und 7,5 ist;
b) der a_{w}-Wert zwischen 0,86 und 0,95, vorzugsweise zwischen 0,88 und 0,92 ist;
c) das Nährmedium ein festes Nährmedium ist, das vorzugsweise Agar enthält;
d) der Hefeextrakt 5-40 Gew.-% Proteine, 5-40 Gew.-% freie Aminosäuren und 0,001-2 Gew.-% Vitamine der B-Gruppe umfasst oder aus diesen besteht; und/oder
e) der Malzextrakt 55-80 Gew.-% Kohlenhydrate, insbesondere Maltose, Glucose, Fructose, Saccharose, 1-12 Gew.-% stickstoffhaltige Verbindungen, insbesondere Peptide, Aminosäuren, Purine, Vitamine, umfasst oder aus Maltose besteht.

4. Verfahren zur Isolierung und/oder Anreicherung von osmotoleranten Mikroorganismen, umfassend:
- Kultivieren einer Probe eines osmotoleranten Mikroorganismus in einem Nährmedium nach einem der Ansprüche 1 bis 3.

5. Verfahren nach Anspruch 4, wobei der osmotolerante Mikroorganismus eine Hefe, vorzugsweise eine Hefe ausgewählt aus der Gattung *Zygosaccharomyces, Schizosaccharomyces, Torulaspora*, *Candida, Pichia, Starmerella*, *Wickerhamomyces* und *Debarymyces* ist.

6. Verfahren nach Anspruch 4 oder 5, wobei die Probe entnommen oder ausgewählt ist aus Nahrungsmitteln, vorzugsweise Flüssigzucker, Marzipan, Persipan, Schokoladenprodukt, Nougat, Honig, Sirup (z. B. Invertzuckersirup, Glucosesirup, Fructosesirup, Melasse), Zuckerdicksaft, Fruchtkonzentrat, Konfitüre, Limonade, Fleischprodukt, Feinkostsalate, Kondensmilch, Trockenfrüchte oder Sojasauce, Futtermitteln, pharmazeutischen Produkten, Kosmetika und klinisch-diagnostischen Proben.

7. Verwendung eines Nährmediums nach einem der Ansprüche 1 bis 3 zur Kultivierung von osmotoleranten Mikroorganismen, vorzugsweise osmotolerante Hefen.

8. Kit zur Untersuchung von osmotoleranten Mikroorganismen, umfassend das Nährmedium nach einem der Ansprüche 1 bis 3, ein Kulturgefäß und eine Vorrichtung zum Nachweis der Gasbildung der osmotoleranten Mikroorganismen.

## Claims

1. Culture medium comprising:
15 to 30 wt.% glucose, preferably 20 to 25 wt.% glucose,
0.7 to 2.0 wt.% fructose, preferably 1.0 to 1.3 wt.% fructose,
0.2 to 0.8 wt.% yeast extract, preferably 0.3 to 0.5 wt.% yeast extract,
0.4 to 1.5 wt.% malt extract, preferably 0.6 to 1.0 wt.% malt extract,
0.2 to 0.8 wt.% peptone, preferably 0.3 to 0.6 wt.% peptone
5 to 15 wt.% glycerol, preferably 8 to 12 wt.% glycerol
2 to 10 wt.% KCl, preferably 3 to 5 wt.% KCl
0.04 to 0.16 wt.% MgSO₄, preferably 0.05 to 0.1 wt.% MgSO₄,
0.04 to 0.32 wt.% (NH₄)₂HPO₄, preferably 0.1 to 0.2 wt.% (NH₄)₂HPO₄
0.04 to 0.16 wt.% (NH₄)₂SO₄, preferably 0.05 to 0.1 wt.% (NH₄)₂SO₄,
0.00003 to 0.0002 wt.% thiamine, preferably 0.00005 to 0.00015 wt.% thiamine, and
42.2 to 76.3 wt.% water, preferably 57 to 67 wt.% water.

2. Culture medium according to claim 1, consisting of:
21.92 wt.% glucose,
1.15 wt.% fructose,
0.38 wt.% yeast extract,
0.77 wt.% malt extract,
0.38 wt.% peptone,
9.69 wt.% glycerol,
3.85 wt.% KCl,
0.08 wt.% MgSO₄,
0.15 wt.% of (NH₄)₂HPO₄,
0.08 wt.% (NH₄)₂SO₄,
0.0001 wt.% thiamine, and
61.54 wt.% water.

3. Culture medium according to any one of claims 1 or 2, wherein
(a) the pH is between 4.5 and 7.5
b) the a_{w} value is between 0.86 and 0.95, preferably between 0.88 and 0.92;
c) the culture medium is a solid culture medium, preferably containing agar;
d) the yeast extract comprises or consists of 5-40 wt.% proteins, 5-40 wt.% free amino acids and 0.001-2 wt.% B group vitamins; and/or
(e) the malt extract comprises 55-80 wt.% carbohydrates, in particular maltose, glucose, fructose, sucrose, 1-12 wt.% nitrogenous compounds, in particular peptides, amino acids, purines, vitamins, or consists of maltose.

4. Method for the isolation and/or enrichment of osmotolerant microorganisms comprising:
- culturing a sample of an osmotolerant microorganism in a culture medium according to any one of claims 1 to 3.

5. Method according to claim 4, wherein the osmotolerant microorganism is a yeast, preferably a yeast selected from the genus *Zygosaccharomyces, Schizosaccharomyces, Torulaspora*, *Candida, Pichia, Starmerella*, *Wickerhamomyces* and *Debarymyces.*

6. Method according to claim 4 or 5, wherein the sample is taken or selected from food, preferably liquid sugar, marzipan, persipan, chocolate product, nougat, honey, syrup (e.g. invert sugar syrup, glucose syrup, fructose syrup, molasses), concentrated sugar, fruit concentrate, jam, lemonade, meat product, deli food salads, condensed milk, dried fruit or soy sauce, animal feed, pharmaceutical products, cosmetics and clinical diagnostic samples.

7. Use of a culture medium according to any one of claims 1 to 3 for the cultivation of osmotolerant microorganisms, preferably osmotolerant yeasts.

8. Kit for examining osmotolerant microorganisms, comprising
the culture medium according to any one of claims 1 to 3, a culture vessel and a device for detecting the gas formation of the osmotolerant microorganisms.

## Revendications

1. Milieu de culture, comprenant :
15 à 30 % en poids de glucose, de préférence 20 à 25 % en poids de glucose,
0,7 à 2,0 % en poids de fructose, de préférence 1,0 à 1,3 % en poids de fructose,
0,2 à 0,8 % en poids d'extrait de levure, de préférence 0,3 à 0,5 % en poids d'extrait de levure,
0,4 à 1,5 % en poids d'extrait de malt, de préférence 0,6 à 1,0 % en poids d'extrait de malt,
0,2 à 0,8 % en poids de peptone, de préférence 0,3 à 0,6 % en poids de peptone,
5 à 15 % en poids de glycérine, de préférence 8 à 12 % en poids de glycérine,
2 à 10 % en poids de KCl, de préférence 3 à 5 % en poids de KCl
0,04 à 0,16 % en poids de MgSO₄, de préférence 0,05 à 0,1 % en poids de MgSO₄,
0,04 à 0,32 % en poids de (NH₄)₂HPO₄, de préférence 0,1 à 0,2 % en poids de (NH₄)₂HPO₄
0,04 à 0,16 % en poids de (NH₄)₂SO₄, de préférence 0,05 à 0,1 % en poids de (NH₄)₂SO₄,
0,00003 à 0,0002 % en poids de thiamine, de préférence et 0,00005 à 0,00015 % en poids de thiamine, et 42,2 à 76,3 % en poids d'eau, de préférence 57 à 67 % en poids d'eau.

2. Milieu de culture selon la revendication 1, se composant de :
21,92 % en poids de glucose,
1,15 % en poids de fructose,
0,38 % en poids d'extrait de levure,
0,77 % en poids d'extrait de malt,
0,38 % en poids de peptone,
9,69 % en poids de glycérine,
3,85 % en poids de KCl,
0,08 % en poids de MgSO₄,
0,15 % en poids de (NH₄)₂HPO₄,
0,08 % en poids de (NH₄)₂SO₄,
0,0001 % en poids de thiamine et
61,54 % en poids d'eau.

3. Milieu de culture selon l'une quelconque des revendications 1 ou 2, dans lequel :
a) le pH est compris entre 4,5 et 7,5 ;
b) la valeur a_{w} est comprise entre 0,86 et 0,95, de préférence entre 0,88 et 0,92 ;
c) le milieu de culture est un milieu de culture solide, contenant de préférence de l'agar ;
d) l'extrait de levure comprend ou est constitué de 5 à 40 % en poids de protéines, 5 à 40 % en poids d'acides aminés libres et 0,001 à 2 % en poids de vitamines du groupe B ; et/ou
e) l'extrait de malt comprend 55 à 80 % en poids d'hydrates de carbone, notamment du maltose, du glucose, du fructose, du saccharose, 1 à 12 % en poids de composés azotés, notamment des peptides, des acides aminés, des purines, des vitamines, ou est constitué de maltose.

4. Procédé d'isolement et/ou d'enrichissement de microorganismes osmotolérants, comprenant :
- la culture d'un échantillon d'un micro-organisme osmotolérant dans un milieu de culture selon l'une quelconque des revendications 1 à 3.

5. Procédé selon la revendication 4, dans lequel le micro-organisme osmotolérant est une levure, de préférence une levure choisie dans le genre *Zygosaccharomyces, Schizosaccharomyces, Torulaspora*, *Candida, Pichia, Starmerella*, *Wickerhamomyces* et *Debarymyces.*

6. Procédé selon la revendication 4 ou 5, dans lequel l'échantillon est prélevé ou sélectionné parmi des denrées alimentaires, de préférence sucre liquide, pâte d'amandes, persipan, produit de chocolat, praliné, miel, sirop (par exemple sirop de sucre inverti, sirop de glucose, sirop de fructose, mélasse), jus de sucre concentré, concentré de fruits, confiture, limonade, produit de viande, salades traiteurs, lait condensé, fruits secs ou sauce de soja, aliments pour animaux, produits pharmaceutiques, cosmétiques et échantillons de diagnostic clinique.

7. Utilisation d'un milieu de culture selon l'une quelconque des revendications 1 à 3 pour la culture de microorganismes osmotolérants, de préférence de levures osmotolérantes.

8. Kit pour l'examen de microorganismes osmotolérants, comprenant :
le milieu de culture selon l'une quelconque des revendications 1 à 3, un récipient de culture et un dispositif pour détecter la formation de gaz des microorganismes osmotolérants.
